(19) Europäisches Patentamt
European Patent Office
Office européen des brevets

(11) Veröffentlichungsnummer: **0 113 092**
**B1**

(12) # EUROPÄISCHE PATENTSCHRIFT

(45) Veröffentlichungstag der Patentschrift: **21.05.86**

(51) Int. Cl.⁴: **C 07 D 215/56, A 61 K 31/495**

(21) Anmeldenummer: **83112724.6**

(22) Anmeldetag: **17.12.83**

(54) **1-Cyclopropyl-6-fluor-1,4-dihydro-4-oxo-7-(4-(oxoalkyl)-1-piperazinyl)-3-chinolincarbonsäuren und ihre Derivate, Verfahren zu ihrer Herstellung sowie diese enthaltende antibakterielle Mittel.**

(30) Priorität: **29.12.82 DE 3248505**

(43) Veröffentlichungstag der Anmeldung: **11.07.84 Patentblatt 84/28**

(45) Bekanntmachung des Hinweises auf die Patenterteilung: **21.05.86 Patentblatt 86/21**

(84) Benannte Vertragsstaaten: **AT BE CH DE FR GB IT LI LU NL SE**

(56) Entgegenhaltungen:
**EP - A - 0 004 279**
**EP - A - 0 049 355**
**EP - A - 0 078 362**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Petersen, Uwe, Dr., Auf dem Forst 4, D-5090 Leverkusen 1 (DE)**
Erfinder: **Grohe, Klaus, Dr., Am Wasserturm 10, D-5068 Odenthal (DE)**
Erfinder: **Zeiler, Hans-Joachim, Dr., Elsbeekerstrasse 46, D-5620 Velbert 15 (DE)**
Erfinder: **Metzger, Karl Georg, Dr., Pahlkestrasse 15, D-5600 Wuppertal 1 (DE)**

**EP 0 113 092 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue 1-Cyclopropyl-6-fluor-1,4-dihydro-4-oxo-7-/4-(oxoalkyl)-1-piperazinyl/-3-chinolincarbonsäuren und deren Derivate, Verfahren zu ihrer Herstellung sowie diese enthaltende antibakterielle Mittel und Futterzusatzmittel.

Es ist bereits bekannt geworden, daß 1-Ethyl-6-fluor-1,4-dihydro-4-oxo-7-(1-piperazinyl)-3-chinolincarbonsäure (Norfloxacin) antibakterielle Eigenschaften besitzt [J. Med. Chem. 23, 1358 (1980)].

Es wurde nun gefunden, daß die neuen 1-Cyclopropyl-6-fluor-1,4-dihydro-4-oxo-7-[4-(oxoalkyl)-1-piperazinyl]-3-chinolincarbonsäuren der Formel (I)

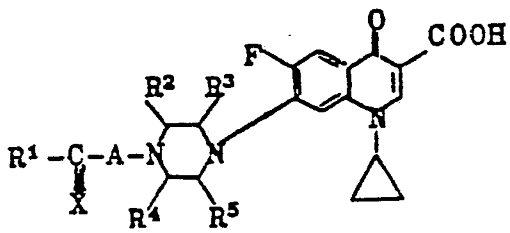

(I)

in der

$R^1$ Wasserstoff, geradkettiges oder verzweigtes Alkyl mit 1 bis 4 Kohlenstoffatomen und gegebenenfalls 1-3 fach durch Trifluormethyl, Methyl, Ethyl, Fluor, Chlor, Brom, Phenyl, Hydroxy oder Aloxy mit 1-4 Kohlenstoffatomen substituiertes Phenyl,

$R^2$, $R^3$, $R^4$ und $R^5$ gleich oder verschieden sein können und Wasserstoff, Methyl, Ethyl, n- oder i-Propyl,

X O, N-O-R', N-NH-R'' und $(OR'')_2$, wobei

R' für Wasserstoff, geradkettiges oder verzweigtes Alkyl mit 1 bis 6 Kohlenstoffatomen, Cycloalkyl mit 5 bis 6 Kohlenstoffatomen, Cycloalkyl mit 5 bis 6 Kohlenstoffatomen, Benzyl, Chlorbenzyl oder Tetrahydropyranyl,

R'' für Methyl, Phenyl, Carbamoyl oder Thiocarbamoyl und

R'' für Methyl und Ethyl oder (OR''')2 für

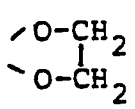

stehen und

A eine Alkylengruppe mit 1 - 4 Kohlenstoffatomen, die gegebenenfalls durch Alkyl mit 1-4 Kohlenstoffatomen oder Phenyl substituiert ist, bedeutet, und deren pharmazeutisch verwendbare Säureadditions-, Alkali- und Erdalkalisalze und Hydrate,

eine hohe antibakterielle Wirkung aufweisen.

Bevorzugt sind diejenigen Verbindungen der Formel (I), in der

$R^1$ Wasserstoff, geradkettiges oder verzweigtes Alkyl mit 1-3 Kohlenstoffatomen oder gegebenenfalls 1-3 fach durch Trifluormethyl, Methyl, Ethyl, Fluor, Chlor, Brom, Phenyl, Hydroxy oder Alkoxy mit 1-4 Kohlenstoffatomen substituiertes Phenyl,

$R^2$, $R^3$, $R^4$ und $R^5$ Wasserstoff, Methyl oder Ethyl,

X O, N-O-R', N-NH-R'' oder $(OR''')_2$ bedeuten, wobei

R' Wasserstoff, Alkyl mit 1-4 Kohlenstoffatomen, Benzyl oder Tetrahydropyranyl,

R'' Carbamoyl oder Thiocarbamoyl,

R''' Methyl oder Ethyl und

A Alkylen mit 1 bis 3 Kohlenstoffatomen

bedeuten, und deren pharmazeutisch verwendbare Säureadditions-, Alkali- und Erdalkalisalze und Hydrate.

Besonders bevorzugt sind diejenigen Verbindungen der Formel (I), in der

$R^1$ Wasserstoff, Alkyl mit 1-2 Kohlenstoffatomen oder gegebenenfalls 1-2 fach durch Trifluormethyl, Methyl, Fluor, Chlor, Hydroxy oder Alkoxy mit 1-2 Kohlenstoffatomen substituiertes Phenyl,

$R^2$ Wasserstoff, Methyl oder Ethyl,

$R^3$ Wasserstoff,

$R^4$ Wasserstoff, Ethyl oder Methyl,

$R^5$ Wasserstoff oder Methyl,

X O, N-O-R', N-NH-R'' oder $(OR''')_2$ bedeuten, wobei

R' Wasserstoff, Alkyl mit 1-2 Kohlenstoffatomen, Benzyl oder Tetrahydropyranyl,
R'' Carbamoyl oder Thiocarbamoyl,
R''' Methyl oder Ethyl und
A Alkylen mit 1-2 Kohlenstoffatomen
bedeuten, und deren pharmazeutisch verwendbare Säureadditions-, Alkali- und Erdalkalisalze und Hydrate.

Weiterhin wurde gefunden, daß man die erfindungsgemäßen Verbindungen der Formel (I) erhält, wenn man eine Verbindung der Formel (II)

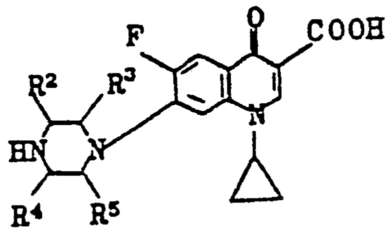

(II)

in welcher
$R^2$, $R^3$, $R^4$ und $R^5$ die oben angegebene Bedeutung haben, mit einer Verbindung der Formel (III)

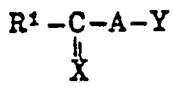

(III)

in welcher
$R^1$, X und A die oben angegebene Bedeutung haben und Y für Halogen, vorzugsweise Chlor oder Brom steht, umsetzt (Methode A).

Die erfindungsgemäßen Verbindungen können auch erhalten werden, indem man Verbindungen der Formel (II)

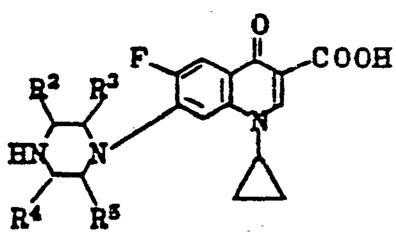

(II)

mit Alkenonen der Formel (IV)

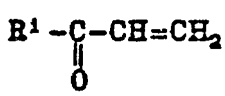

(IV)

in welcher
$R^1$ die oben angegebene Bedeutung hat, zu den erfindungsgemäßen Verbindungen der Formel (Ia) = (I; mit X = O, A = $-CH_2CH_2-$)

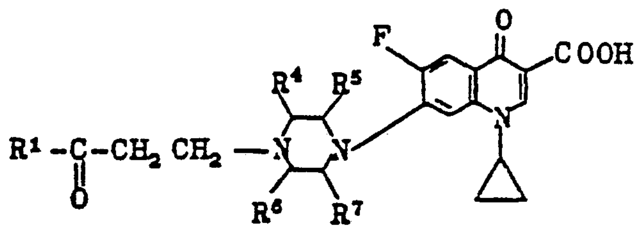

(Ia)

umsetzt (Methode B).

Die erfindungsgemäßen Verbindungen können außerdem erhalten werden, indem man Verbindungen der Formel (V)

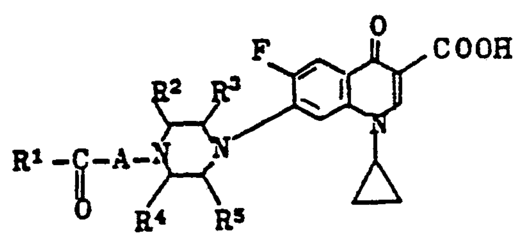

(V)

in welcher $R^1$, $R^2$, $R^3$, $R^4$, $R^5$ und A die oben angegebenen Bedeutungen haben, mit Verbindungen der Formel (VIa) beziehungsweise (VIb)

$H_2N$-O-R' (VIa)

$H_2N$-NH-R'' (VIb)

in welcher R' und R'' die oben angegebenen Bedeutungen besitzen, zu den Verbindungen der Formel (Ib) (I; mit X = N-0-R') beziehungsweise (Ic) (I; mit X = NH-R'')

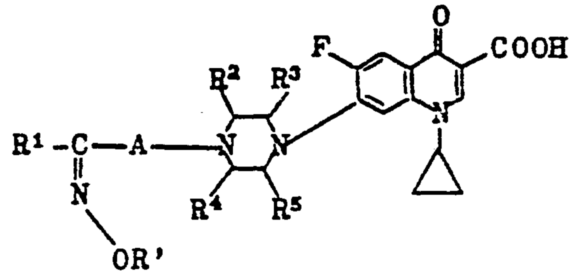

(Ib)

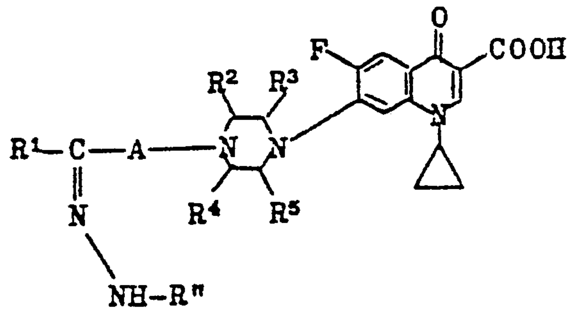

(Ic)

kondensiert (Methode C). Hierbei können die Verbindungen (VIa) beziehungsweise (VIb) auch in Form der Hydrochloride oder Sulfate eingesetzt werden.

Überraschenderweise zeigen die erfindungsgemäßen 1-Cyclo-propyl-6-fluor-1,4-dihydro-4-oxo-7-[4-(oxoalkyl)-1-piperazinyl] -3-chinolincarbonsäuren und ihre Derivate eine erheblich höhere antibakterielle Wirkung als die nach dem Stand der Technik bekannte 1-Ethyl-6-fluor-1,4-dihydro-4-oxo-7-(1-piperazinyl)-3-chinolincarbonsäure. Sie eignen sich daher als Wirkstoffe für die Human- und Veterinärmedizin, wobei zur Veterinärmedizin auch die Vorbeugung und Behandlung von Fischen zu zählen ist.

Die erfindungsgemäßen Stoffe stellen somit eine Bereicherung der Pharmazie dar.

Verwendet man beispielsweise bei der Umsetzung von (II) mit (III) nach der Methode A 1-Cyclopropyl-6-fluor-1,4-dihydro-4-oxo-7-(1-piperazinyl)-3-chinolincarbonsäure und Chloraceton als Ausgangsverbindungen, so kann der Reaktionsablauf durch das folgende Formelschema wiedergegeben werden:

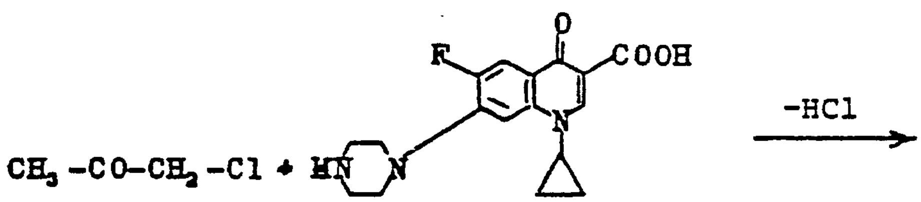


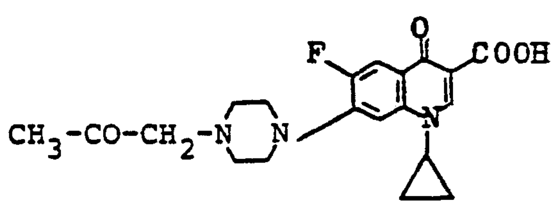

Verwendet man beispielsweise bei der Umsetzung von (II) mit (IV) nach der Methode B 1-Cyclopropyl-6-fluor-1,4-dihydro-4-oxo-7-(1-piperazinyl)-3-chinolincarbonsäure und Methyl-vinyl-keton als Ausgangssubstanzen, so kann der Reaktionsablauf durch das folgende Formelschema wiedergegeben werden.

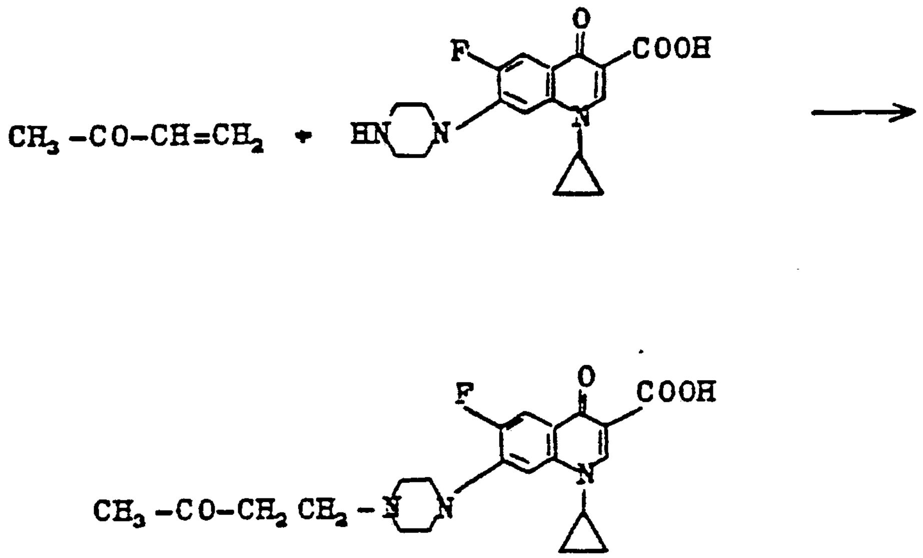

Verwendet man beispielsweise bei der Umsetzung von (V) mit (VI) nach der Methode C 1-Cyclopropyl-6-fluor-1,4-di-hydro-4-oxo-7-[4-(2-oxopropyl)-1-piperazinyl]-3-chinolincarbonsäure und Methoxyaminhydrochlorid als Ausgangsverbindungen, so kann der Reaktionsablauf durch das folgende Formelschema wiedergegeben werden:

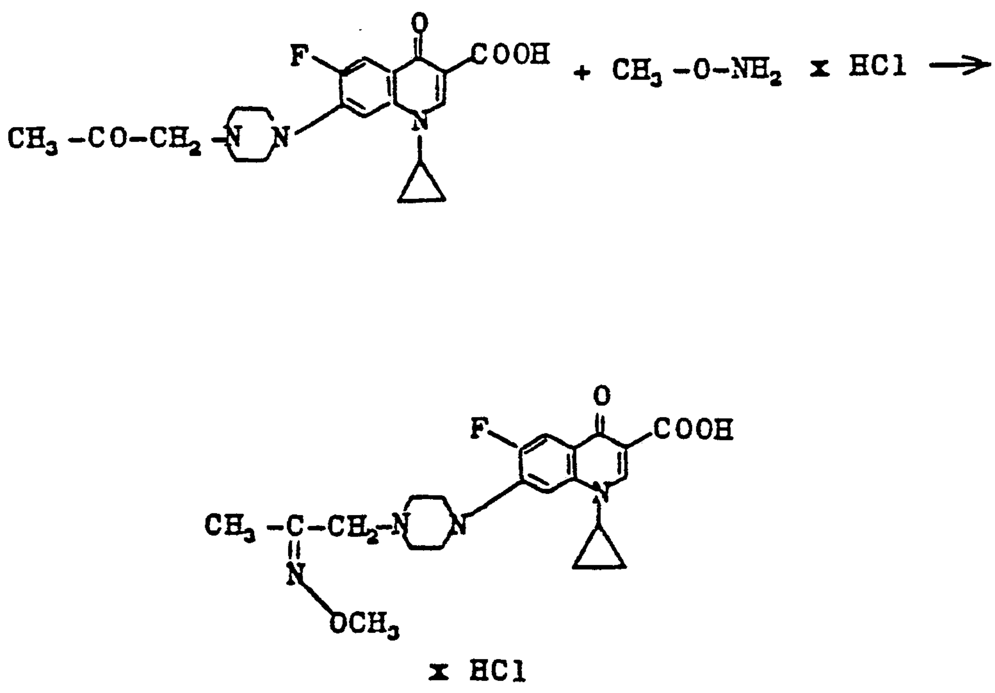

Die als Ausgangsverbindungen verwendeten 1-Cyclopropyl-6-fluor-1,4-dihydro-4-oxo-7-(1-piperazinyl)-3-chinolincarbonsäuren der Formel (II) können durch Umsetzung von 7-Chlor-1-cyclopropyl-6-fluor-1,4-dihydro-4-oxo-3-chinolincarbonsäure der Formel (VII)

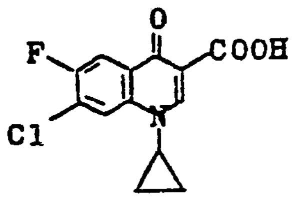

(VII)

mit Piperazin- oder Piperazinderivaten der Formel (VIII)

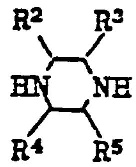

(VIII)

in welcher $R^1$, $R^2$, $R^3$ und $R^4$ die oben angegebene Bedeutung haben, hergestellt werden. Man arbeitet hierbei in einem Verdünnungsmittel wie Dimethylsulfoxid, Hexamethylphosphorsäuretrisamid, Sulfolan, Wasser, einem Alkohol oder Pyridin bei Temperaturen von 20 - 200°C, vorzugsweise bei 80 - 180°C. Bei der Durchführung des Verfahrens setzt man auf 1 Mol Carbonsäure (VII) 1-15 Mol der Verbindung (VIII), vorzugsweise 1-6 Mol der Verbindung (VIII) ein. Bei Verwendung äquivalenter Mengen der Carbonsäure (VII) und des Piperazinderivates (VIII) führt man die Umsetzung in Gegenwart eines säurebindenden Mittels, beispielsweise Triethylamin, 1,4-Diaza-bicyclo-[2,2,2]octan oder 1,8-Diaza-bicyclo[5,4,0]undec-7-en durch.

Als Beispiele für die auf diese Weise herstellbaren Ausgangsprodukte der Formel (II) seien genannt:

1-Cyclopropyl-6-fluor-1,4-dihydro-4-oxo-7-(1-piperazinyl)-3-chinolincarbonsäure,
1-Cyclopropyl-6-fluor-1,4-dihydro-4-oxo-7-(2,5-dimethyl-1-piperazinyl)-3-chinolincarbonsäure,
1-Cyclopropyl-6-fluor-1,4-dihydro-4-oxo-7-(3,5-dimethyl-1-piperazinyl)-3-chinolincarbonsäure,
1-Cyclopropyl-6-fluor-1,4-dihydro-4-oxo-7-(3-methyl-1-piperazinyl)-3-chinolincarbonsäure,
1-Cyclopropyl-6-fluor-1,4-dihydro-4-oxo-7-(3-ethyl-1-pi-perazinyl)-3-chinolincarbonsäure,
1-Cyclopropyl-6-fluor-1,4-dihydro-4-oxo-7-(3,5-diethyl-1-piperazinyl)-3-chinolincarbonsäure,
1-Cyclopropyl-6-fluor-1,4-dihydro-4-oxo-7-(2,3,5-tri-methyl-1-piperazinyl)-3-chinolincarbonsäure,
1-Cyclopropyl-6-fluor-1,4-dihydro-4-oxo-7-(2,3,5,6-te-tramethyl-1-piperazinyl)-3-chinolincarbonsäure.

Die als Zwischenprodukt verwendete 7-Chlor-1-cyclopropyl-6-fluor-1,4-dihydro-4-oxo-3-chinolincarbonsäure der Formel (VII) kann gemäß folgendem Reaktionsschema hergestellt werden.

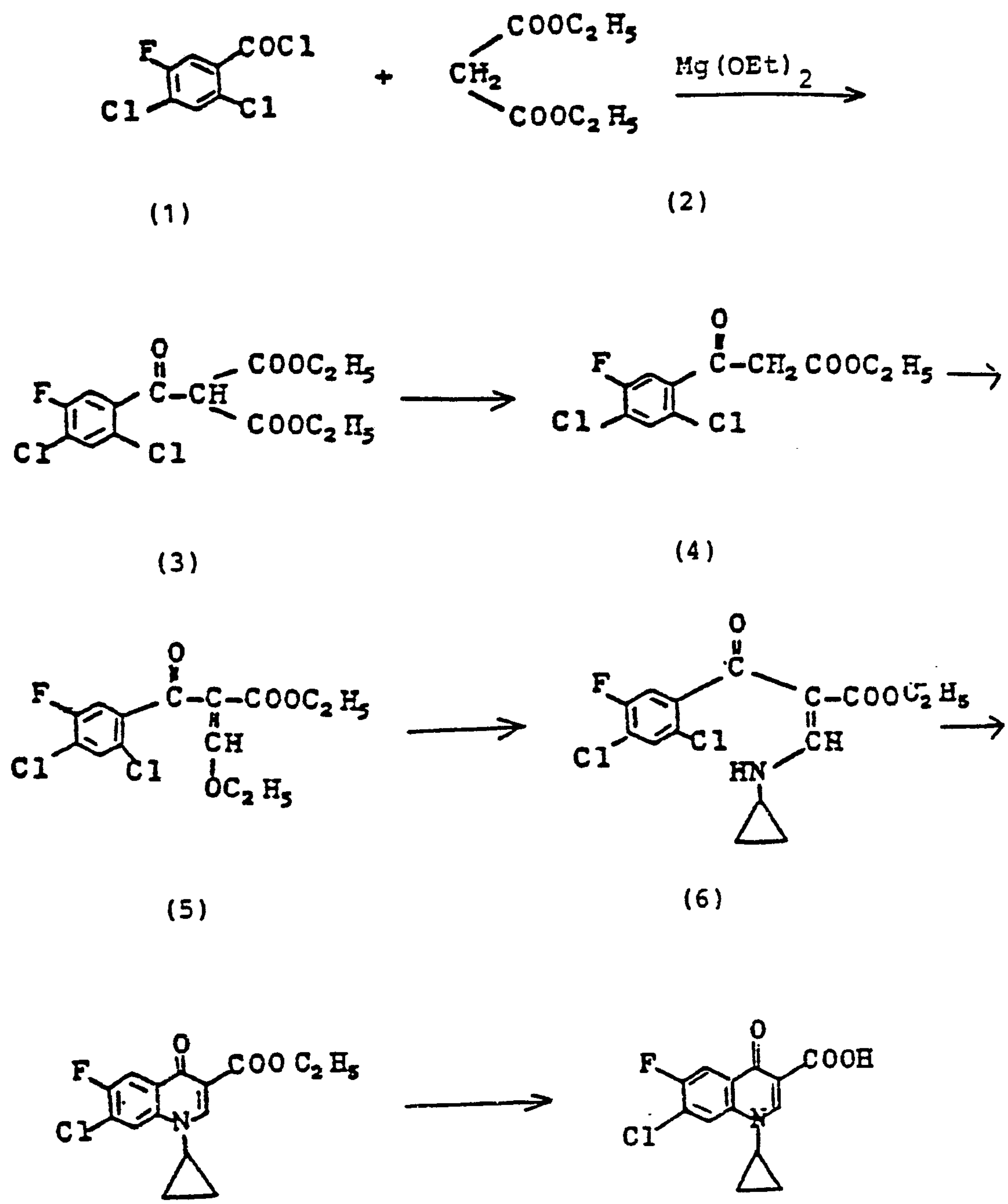


(1) (2)

(3) (4)

(5) (6)

(7) (VII)

Danach wird Malonsäurediethylester (2) mit (1) in Gegenwart von Magnesiumalkoholat mit 2,4-Dichlor-5-fluorbenzoylchlorid (Deutsche Patentanmeldung Nr. 3 142 856.8) zum Acylmalonester (3) acyliert (Organikum, 3. Aufl. 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Aroylessigsäureethylester (4), der mit o-Ameisensäuretriethylester/Acetanhydrid in den 2-(2,4-Dichlor-5-fluor-benzoyl)-3-ethoxy-acrylsäureethylester (5) übergeht. Die Umsetzung von (5) mit Cyclopropylamin in einem Lösungsmittel, wie z.B. Methylenchlorid, Alkohol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300°C, bevorzugt 80 bis 180°C durchgeführt.

Als Verdünnungsmittel können Dioxan, Dimethylsulfoxid, N-Methyl-pyrrolidon, Sulfolan, Hexamethylphosphorsäuretriamid und bevorzugt N,N-Dimethylformamid verwendet werden.

Als Säurebinder kommen für diese Reaktionsstufe Kaliumtert.-butanolat, Butyl-lithium, Lithium-phenyl, Phenylmagnesiumbromid, Natriummethylat, Natriumhydrid und besonders bevorzugt Kalium- oder Natriumcarbonat in Betracht. Es kann vorteilhaft sein, einen Überschuß von 10 Mol-% an Base einzusetzen.

Die in dem letzten Schritt erfolgende Esterhydrolyse unter basischen oder sauren Bedingungen führt zur 7-Chlor-1-cyclopropyl-6-fluor-1,4-dihydro-4-oxo-3-chinolincarbon-säure VII.

Das als Ausgangsmaterial für diesen Syntheseweg verwendete 2,4-Dichlor-5-fluor-benzoylchlorid (1) und die entsprechende Carbonsäure sowie das für die Herstellung von (1) benötigte 3-Fluor-4,6-dichlortoluol (10) sind noch nicht bekannt.

Das nachstehende Formelschema zeigt die Herstellung dieser Vor- bzw. Zwischenprodukte, ausgehend von 2,4-Dichlor-5-methyl-anilin (8).

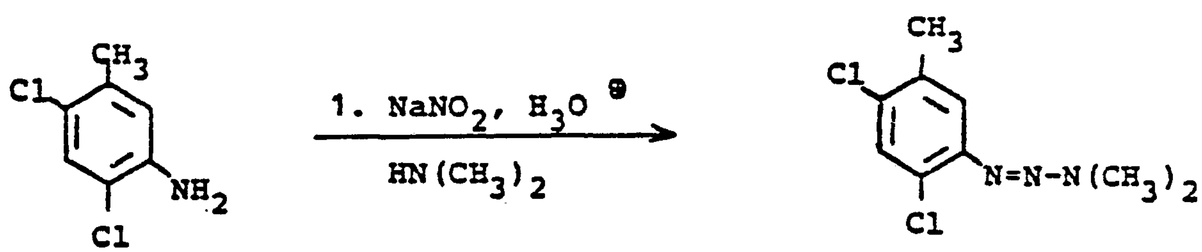


(8) (9)

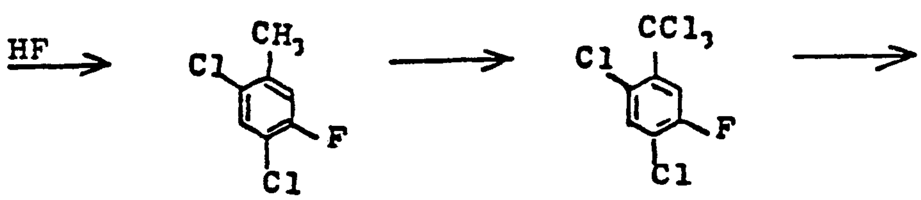


(10) (11)

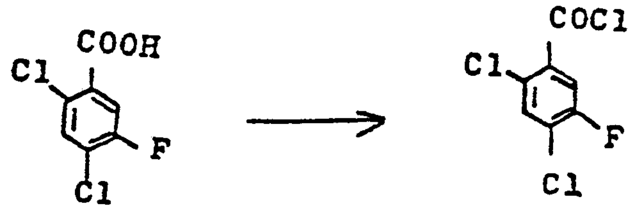

(12) (1)

Danach wird 2,4-Dichlor-5-methyl-anilin (8) mit Hilfe von $NaNO_2$ diazotiert und das dabei entstehende Diazoniumsalz mit Dimethylamin in das Triazen (9) übergeführt.

Das Triazen (9) wird in überschüssiger wasserfreier HF gelöst. Dabei spaltet das Triazen in 2,4-Dichlor-5-methyl-diazoniumfluorid und Dimethylamin. Ohne Zwischenisolierung wird diese Lösung thermisch bei 130-140°C unter $N_2$-Abspaltung in 3-Fluor-4,6-dichlortoluol (10) gespalten. Ausbeute: 77,7 % der Theorie.

Das 3-Fluor-4,6-dichlortoluol (10) wird in einem Temperaturbereich von 110 bis 160°C unte UV-Bestrahlung zu 2,4-Dichlor-5-fluor-1-trichlormethylbenzol (11) chloriert.

Die Verseifung von (11) mit 95 %iger Schwefelsäure führt zu 2,4-Dichlor-5-fluor-benzoesäure (12), die mit Thionylchlorid in das Carbonsäurechlorid (1) (Sdp. 121°C/20 mbar; $n_D^{20}$ 1,5722) übergeht.

Die erfindungsgemäß verwendbaren Verbindungen der Formel (III) sind bereits bekannt oder können nach allgemein bekannten Verfahren erhalten werden. Als Beispiele seien genannt:

Chloracetaldehyd, Chloraceton, Bromaceton, 1-Chlorbuta-non, 3-Chlorbutanon, 1-Chlor-3,3-dimethylbutanon, 1-Chlor-2,2-dimethylpropanon, 1-Chlor-2-hexanon, 5-Brom-2-penta-non, 2-Brom-3-pentanon, 4-Brom-2-pentanon, Phenacylchlorid, 2,4-Dihydroxyphenacylchlorid, 3,4-Dihydroxyphenacyl-chlorid, 2-Hydroxy-4-methoxy-phenacylchlorid, 4-Fluor-phenacylchlorid, 4-Chlorphenacylchlorid, 2,4-Dichlor-phenacylchlorid, 3-Bromphenacylbromid, 4-Methylphenacyl-bromid, Bromacetaldehyddimethylacetal, Chloracetaldehydroxim-0-methylether, 2-Chlormethyl-2-tert.-butyldioxolan, 2-Brommethyl-dioxolan.

Die erfindungsgemäß verwendbaren Alkenone der Formel (IV) sind bereits bekannt. Als Beispiele seien genannt:

Acrolein, Methyl-vinyl-keton, Ethyl-vinyl-keton, Isopropyl-vinyl-keton, n-Propyl-vinyl-keton, n-Butyl-vinyl-keton, Isobutyl-vinyl-keton, sek.-Butyl-vinyl-keton, tert.-Butyl-vinyl-keton, 4-Methyl-3-penten-2-on, Methyl-(2-phenylvinyl)-keton.

Die erfindungsgemäß verwendbaren Hydroxylamine der Formel (VIa) sind ebenfalls bekannt.

Als Beispiele seien genannt:

Hydroxylamin, Methoxyamin, Ethoxyamin, n-Propoxyamin, tert.-Butoxyamin, n-Pentyloxyamin, Cyclohexyloxyamin, 2-Tetrahydropyranyloxyamin, Benzyloxyamin, 4-Chlorbenzyl-oxyamin, 2,6-Dichlorbenzyloxyamin.

Die erfindungsgemäß verwendbaren Hydrazinderivate der Formel (VIb) sind bereits bekannt. Als Beispiele seien genannt:

Methylhydrazin, Phenylhydrazin, Semicarbazid, Thiosemicarbazid.

Die Umsetzung von (II) mit (III) (Methode A) wird vorzugsweise in einem Verdünnungsmittel wie Dimethylsulfoxid, N,N-Dimethylformamid, Hexamethylphosphorsäuretrisamid, Sulfolan, Dioxan, Pyridin oder einem Alkohol bei Temperaturen von 20 - 180 °C, vorzugsweise 40 - 110°C, vorgenommen.:

Die Umsetzung kann bei Normaldruck, aber auch bei erhöhtem Druck, durchgeführt werden. Im allgemeinen arbeitet man bei Drucken zwischen etwa 1 und 10 bar.

Als Säurebinder können alle üblichen anorganischen und organischen Säurebindungsmittel verwendet werden. Hierzu gehören vorzugsweise die Alkalihydroxide, Alkalicarbonate, Pyridin und tert.-Amine wie Triethylamin, 1,4-Diazabicy-clo[2,2,2]octan (DABCO).

Bei der Durchführung des erfindungsgemäßen Verfahrens setzt man auf 1 Mol der Verbindung (II) 1 bis 4 Mol, vorzugsweise 1 bis 1,5 Mol der Verbindung (III) ein.

Die Umsetzung von (II) mit (IV) (Methode B) wird vorzugsweise in einem Verdünnungsmittel wie Dioxan, Dimethylsulfoxid, N,N-Dimethylformamid, Methanol, Ethanol, Propanol oder Isopropanol durchgeführt.

Die Reaktionstemperaturen können in einem größeren Bereich variiert werden. Im allgemeinen arbeitet man zwischen etwa 30 und etwa 150°C, vorzugsweise zwischen 50 und 110°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Verbindung (II) 1-5 Mol, vorzugsweise 1-2 Mol, der Verbindung (IV) ein.

Die Umsetzung von (V) mit (VIa) beziehungsweise (VIb) (Methode C) wird in einem Verdünnungsmittel wie Wasser, Methanol, Ethanol, n-Propanol, Isopropanol, Glykolmonomethylether, Dioxan, N,N-Dimethylformamid, Dimethylsulfoxid oder auch in Mischungen dieser Lösungsmittel durchgeführt. Als Katalysatoren können

anorganische oder organische Säuren wie Salzsäure, Schwefelsäure, Phosphorsäure, 4-Toluolsulfonsäure und Salze wie Natriumacetat oder Kaliumacetat verwendet werden. Die Reaktionstemperaturen können in einem größeren Bereich variiert werden. Im allgemeinen arbeitet man zwischen 40 und etwa 150°C, vorzugsweise zwischen 60 und 100°C.

Die Umsetzung kann bei Normaldruck, aber auch bei erhöhtem Druck, durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Verbindung (V) 1 bis 3 Mol, vorzugsweise 1 bis 1,3 Mol, der Verbindung (VIa) beziehungsweise (VIb) ein.

Als neue antibakterielle Wirkstoffe seien im einzelnen genannt:

7-[4-(2-Oxopropyl)-1-piperazinyl]-1-cyclopropyl-6-fluor-1,4-dihydro-4-oxo-3-chinolincarbonsäure,
7-[4-(3-Oxobutyl)-1-piperazinyl]-1-cyclopropyl-6-fluor-1,4-dihydro-4-oxo-3-chinolincarbonsäure,
7-[4-(3,3-Dimethyl-2-oxobutyl)-1-piperazinyl]-1-cyclo-propyl-6-fluor-1,4-dihydro-4-oxo-3-chinolincarbonsäure,
7-[4-(1'1-Dimethyl-2-oxopropyl)-1-piperazinyl]-1-cyclo-propyl-6-fluor-1,4-dihydro-4-oxo-3-chinolincarbonsäure,
7-[4-Phenacyl-1-piperazinyl]-1-cyclopropyl-6-fluor-1,4-dihydro-4-oxo-3-chinolincarbonsäure,
7-[4-(2,4-Dihydroxyphenacyl)i-piperazinyl]-1-cyclopropyl -6-fluor-1,4-dihydro-4-oxo-3-chinolincarbonsäure,
7-[4-(3,4-Dihydroxyphenacyl)-1-piperazinyl]-1-cyclopropyl -6-fluor-1,4-dihydro-4-oxo-3-chinolincarbonsäure,
7-[4-(2-Hydroxy-4-methoxyphenacyl)-1-piperazinyl]-1-cyclopropyl-6-fluor-1,4-dihydro-4-oxo-3-chinolincarbonsäure,
7-[4-(4-Fluorphenacyl)-1-piperazinyl]-1-cyclopropyl-6-fluor-1,4-dihydro-4-oxo-3-chinolincarbonsäure,
7-[4-(4-Chlorphenacyl)-1-piperazinyl]-1-cyclopropyl-7-fluor-1,4-dihydro-4-oxo-3-chinolincarbonsäure,
7-[4-(2-Hydroximinoethyl)-1-piperazinyl]-1-cyclopropyl-6-fluor-1,4-dihydro-4-oxo-3-chinolincarbonsäure,
7-[4-(2-Methoximinopropyl)-1-piperazinyl]-1-cyclopropyl-6-fluor-1,4-dihydro-4-oxo-chinolincarbonsäure,
7-[4-(2-Benzyloximinopropyl)-1-piperazinyl]-1-cyclopropyl -6-fluor-1,4-dihydro-4-oxo-3-chinolincarbonsäure,
7- {4-[2-(2-Tetrahydropyranyloximino)-propyl]-1-pipera-zinyl {-1-cyclopropyl-6-fluor-1,4-dihydro-4-oxo-3-chinolincarbonsäure,
7-[4-(3-Oximinobutyl)-1-piperazinyl]-1-cyclopropyl-6-fluor-1,4-dihydro-4-oxo-3-chinolincarbonsäure,
7-[4-(2,2-Diethoxyethyl)-1-piperazinyl]-1-cyclopropyl-6-fluor-1,4-dihydro-4-oxo-3-chinolincarbonsäure,
7-[3-Methyl-4-(2-oxopropyl)-1-piperazinyl]-1-cyclopropyl-6-fluor-1,4-dihydro-4-oxo-3-chinolincarbonsäure,
7-[3-Methyl-4-(3-oxobutyl)-1-piperazinyl]-1-cyclopropyl-6-fluor-1,4-dihydro-4-oxo-3-chinolincarbonsäure,
7-[2,5-Dimethyl-4-(2-oxopropyl)-1-piperazinyl]-1-cyclopropyl -6-fluor-1,4-dihydro-4-oxo-3-chinolincarbonsäure,
7-[2,5-Dimethyl-4-(3-oxobutyl)-1-piperazinyl]-1-cyclopropyl -6-fluor-1,4-dihydro-4-oxo-3-chinolincarbonsäure,
7-[3,5-Dimethyl-4-(2-oxopropyl)-1-piperazinyl]-1-cyclopropyl -6-fluor-1,4-dihydro-4-oxo-3-chinolincarbonsäure,
7-[3,5-Dimethyl-4-(3-oxobutyl)-1-piperazinyl]-1-cyclopropyl -6-fluor-1,4-dihydro-4-oxo-3-chinolincarbonsäure.

Die so erhaltenen erfindungsgemäßen Verbindungen der Formel (I) können gegebenenfalls mit einer organischen oder anorganischen Säure in ein Salz übergeführt werden. Zur Salzbildung geeignete Säuren sind z.B. Halogenwasserstoffsäuren wie Salzsäure, Bromwasserstoffsäure, Iodwasserstoffsäure, Schwefelsäure, Essigsäure, Citronensäure, Ascorbinsäure und Benzolsulfonsäure. Als Alkali- beziehungsweise Erdalkalisalze sind vorzugsweise Natrium-, Kalium-, Calcium- und Magnesiumsalze geeignet.

Die erfindungsgemäßen Verbindungen zeigen ein breites antibakterielles Spektrum gegen gram-positive und gramnegative Keime, insbesondere gegen Enterobakteriaceen, vor allem auch gegen solche, die resistent sind gegen verschiedene Antibiotika wie z.B. Penicilline, Cephalosporine, Aminoglykoside, Sulfonamide, Tetracyline.;

Die erfindungsgemäßen Verbindungen weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und; bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, (Staph. = Staphylococcus), Lactobacteriaceae, wie Streptokokken, z.B. Streptococcus pyogenes, $\alpha$ - bzw. ß-hämolysierende Streptokokken, nicht ($\gamma$-)-hämolysierende Streptokokken, Enterokokken und Diplococcus pneumoniae (Pneumokokken) (Str. = Streptococcus); Enterobacteriaceae, wie Escherichiae-Bakterien der Coli-Gruppe: Escherichia-Bakterien, z.B. Escherichia coli, Enterobacter-Bakterien, z.B. E.aerogenes, E.cloacae, Klebsiella-Bakterien, z.B. K-pneumoniae, Serratia, z.B. Serratia marcescens (E. = Enterobacter) (K. =

Klebsiella), Proteae-Bakterien der Proteus-Gruppe: Proteus, z.B. Proteus vulgaris, Pr. morganii, Pr. rettgeri, Pr. mirabilis, (Pr. = Proteus):

Pseudomonadaceae, wie Pseudomonas-Bakterien, A.B. Pseudomonas aeruginosa, (PS. = Pseudomonas);

Bacteroidaceae, wie Bacteroides-Bakterien, z.B. Bacteroides fragilis, (B. = Bacteroides).

Mykoplasmen, z.B. Mykoplasme pneumoniae.

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen, septische Erkrankungen.

Zur Erfindung gehören pharmazeutische Zubereitungen, die neben nichttoxischen, inerten pharmazeutisch geegneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, sie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffen enthalten, z.B. Polyethylenglykole, Fette, z.B. Kakaofett und höhere Ester (z.B. $C_{14}$-Alkohol mit $C_{16}$-Fettsäure) oder Gemische dieser Stoffe. Salben, Pasten, Cremes und Gele können neben dem oder den Wirkstoffen die üblichen Trägerstoffe enthalten z.B. tierische und pflanzliche Fette, Wachse Paraffine Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutiostonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusatze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95

Gewichtsprozent der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/ oder rectal, vorzugsweise oral oder parenteral wie intravenös oder intramuskulär appliziert werden.

Im allgemeinen hat es sich sowohl in der Human- als auch in der Veterinärmedizin als virteilhaft erweisen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder grampositive Bakterien verhindert, gebesssert und/ oder geheilt werden und dadurch eine Förderung des Wachstums und einer Verbesserung der Verwendung des Futters erreicht werden.

Die in den nachfolgenden Beispielen angegebenen $R_f$-Werte wurden auf Kieselgel 60-Fertigplatten (MERCK/Darmstadt) mit Methylenchlorid/Methanol/17 % wäßr. Ammoniak (70/8/1) als Laufmittel gemessen.

**Herstellungsbeispiele für die Ausgangsverbindungen:**

**Beispiel A**

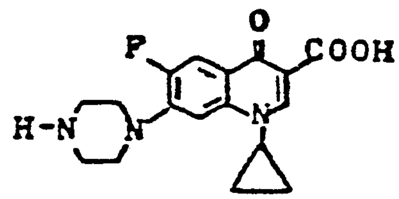

Ein Gemisch von 19,7 g 7-Chlor-1-cyclopropyl-6-fluor-1,4-dihydro-4-oxo-3-chinolincarbonsäure, 30,1 g wasserfreiem Piperazin und 100 ml Dimethylsulfoxid wird 2 Stundenauf 135-140°C erhitzt. Das Lösungsmittel wird im Feinvakuum abdestilliert, der Rückstand in $H_2O$ suspendiert, abgesaugt und mit Wasser gewaschen. Zur weiteren Reinigung wird das feuchte Rohprodukt mit 100 ml Wasser aufgekocht, bei Raumtemperatur abgesaugt, mit $H_2O$ gewaschen und im Vakuumtrockenschrank über $CaC1_2$ bei 100°C bis zur Gewichtskonstanz getrocknet. Man erhält 19,6 g 1-Cyclopropyl-6-fluor-1,4-dihydro-4-oxo-7-(piperazinyl)-3-chinolincarbonsäu-re vom Zersetzungspunkt 255-257°C; $R_F$-Wert: 0,07.

Die als Ausgangsmaterial verwendete 7-Chlor-1-cyclopropyl-6-fluor-1,4-dihydro-4-oxo-3-chinolincarbonsäuren (VII) wird wie folgt hergestellt:

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml abs. Ethanol und 400 ml wasserfreiem Ether zu, wobei ein heftiger Rückfluß zu beobachten ist. Nach dem Abklingen der Reaktion wird noch 2 h zum Sieden erhitzt, mit Trockeneis/Aceton auf −5 bis −10°C gekühlt und bei dieser Temperatur eine Lösung von 227,5 g 2,4-Dichlor-5-fluor-benzoylchlorid (1) in 100 ml abs. Ether langsam zugetropft. Man rührt 1 h bei 0 bis −5°C, läßt über Nacht auf Raumtemperatur kommen und läßt unter Eiskühlung ein Gemisch von 400 ml Eiswasser und 25 ml konz. Schwefelsäure zulaufen. Die Phasen werden getrennt und zweimal mit Ether nachextrahiert. Die vereinigten Etherlösungen werden mit gesättigter NaCl-Lösung gewaschen, mit $Na_2SO_4$ cetrocknet und das Lösungsmittel i. Vak. abgezogen. Man erhält 349,5 g 2,4-Dichlor-5-fluor-benzoyl-malonsäurediethylester (3) als Rohprodukt.

Eine Emulsion von 34,9 g rohem 2,4-Dichlor-5-fluor-ben-zoyl-malonsäurediethylester (3) in 50 ml Wasser wird mit 0,15 g p-Toluolsulfonsäure versetzt. Man erhitzt unter gutem Rühren 3 h zum Sieden, extrahiert die erkaltete Emulsion mehrmals mit Methylenchlorid, wäscht die vereinigten $CH_2Cl_2$-Lösungen einmal mit gesättigter NaCl-Lösung, trocknet mit $Na_2SO_4$ und destilliert das Lösungsmittel i. Vak. ab. Die Fraktionierung des Rückstands i. Feinvak. liefert 21,8 g 2,4-Dichlor-5-fluor-benzoyl-essigsäureethylester (4) vom Sdp. 127-142°C/0,09 mbar.

Ein Gemisch von 21,1 g 2,4-Dichlor-5-fluor-benzoyl-essig-säureethylester (4), 16,65 g o-Ameisensäureethylester und 18,55 g Acetanhydrid wird 2 h auf 150°C erhitzt Dann werden im Wasserstrahlvakuum und zuletzt im Feinvakuum bei einer Badtemperatur von 120°C die flüchtigen Bestandteile abdestilliert. Zurück bleiben 25,2 g roher 2-(2,4-Dichlor-5-benzoyl)-3-ethoxy-acrylsäureethylester (5). Er ist

genügend rein für die weiteren Umsetzungen.

Eine Lösung von 24,9 g 2-(2,4-Dichlor-5-fluor-benzoyl)-3-ethoxy-acrylsäureethylester (5) in 80 ml Ethanol wird unter Eiskühlung und Rühren tropfenweise mit 4,3 g Cyclopropylamin versetzt. Wenn die exotherme Reaktion abgeklungen ist, wird noch 1 h bei RT gerührt, das Lösungsmittel i. Vak. abgezogen und der Rückstand aus Cyclohexan/ Petrolether umkristallisiert. Man erhält 22,9 g 2-(2,4-Dichlor-5-fluor-benzoyl)-3-cyclopropylamino-acrylsäureethylester (6) vom Schmp. 89-90° C.

Eine Lösung von 31,9 g 2-(2,4-Dichlor-5-fluor-benzoyl)-3-cyclopropylamino-acrylsäureethylester (6) in 100 ml wasserfreiem Dioxan wird unter Eiskühlung und Rühren portionsweise mit 3,44 g 80 proz. Natriumhydrid versetzt. Dann wird 30 Min. bei Raumtemperatur und 2 h unter Rückfluß gerührt und das Dioxan i.Vak. abgezogen. Der Rückstand (40,3 g) wird in 150 ml Wasser suspendiert, mit 6,65 g Ätzkali versetzt und 1,5 h refluxiert. Man filtriert die warme Lösung und wäscht mit $H_20$ nach. Dann wird mit halbkonz. Salzsäure unter Eiskühlung auf pH = 1-2 angesauert, der Niederschlag abgesaugt, mit Wasser gewaschen und i.Vak. bei 100° C getrocknet. Man erhält auf diese Weise 27,7 g 7-Chlor-1-cyclopropyl-6-fluor-1,4-dihydro-4-oxo-3-chinolincarbonsäure (VII) vom Schmp. 234-237° C.

**Beispiel B**

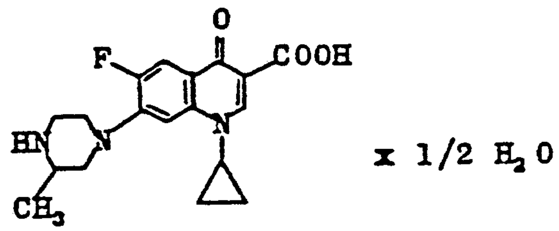

Ein Gemisch aus 2,8 g (0,01 Mol) 7-Chlor-1-cyclopropyl-6-fluor-1,4-dihydro-4-oxo-3-chinolincarbonsäure und 5,1 g (0,051 Mol) 2-Methylpiperazin in 6 ml Dimethylsulfoxid wird 2 Stunden auf 140° C erhitzt. Anschließend wird das Lösungsmittel im Hochvakuum abdestilliert, der Rückstand mit 6 ml heißem Wasser versetzt und 1 Stunde auf 95° C gehalten. Man kühlt mit Eis, saugt den ausgefallenen Niederschlag ab, wäscht mit etwas Wasser und löst ihn in einer Mischung von 0,8 ml Essigsäure und 10 ml Wasser bei 90-100° C auf. Das Filtrat wird mit Kalilauge (0,75 g KOH in 0,7 ml Wasser) auf pH 8 gebracht und der ausgefallene Niederschlag aus Methanol umkristallisiert. Man erhält 1,8 g (52 % d.Th.) 1-Cyclopropyl-6-fluor-1,4-dihydro-4-oxo-7-(3-methyl-1-piperazinyl)-3-chinolincarbonsäure-semihydrat mit einem Zersetzungspunkt von 230-232° C. $R_F$-Wert: 0,11.

Die folgenden Beispiele erläutern die Herstellung der erfindungsgemäßen Verbindungen:

**Beispiel 1**

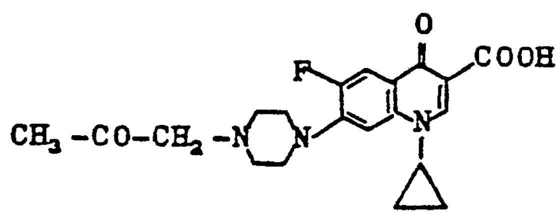

Eine Mischung von 23,2 g (0,07 Mol) 1-Cyclopropyl-6-fluor-1,4-dihydro-4-oxo-7-(1-piperazinyl)-3-chinolincarbonsäure und 9,8 g Chloraceton wird in 350 ml Dimethylformamid mit 14,7 g Triethylamin 3 Stunden auf 80° C erhitzt. Man engt im Hochvakuum ein, verrührt den Rückstand mit 140 ml Wasser und kristallisiert den ungelösten Feststoff aus Glykolmonomethylether um. Ausbeute: 17 g (62,8 % d.Th.) 1-Cyclopropyl-6-fluor-1,4-dihydro-4-oxo-7-/4-(2-oxopro-pyl)-1-piperazinyl/-3-chinolincarbonsäure mit einem Zersetzungspunkt von 220-225° C, $R_f$-Wert= 0,15.

Analog Beispiel 1 werden die folgenden Verbindungen hergestellt:

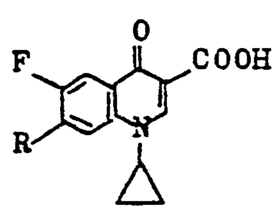

| Beispiel | R | Schmelzpunkt, °C | $R_f$-Wert |
|---|---|---|---|
| 2 | 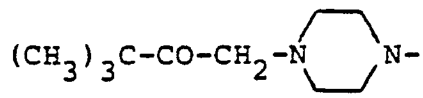 | 207-210°C | 0,22 |
| 3 | 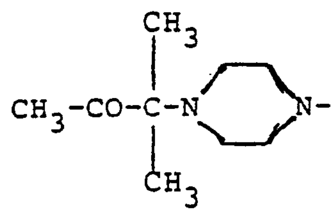 | 268-271°C | 0,29 |
| 4 | 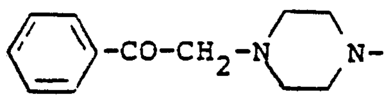 | 198-202°C | 0,32 |
| 5 | 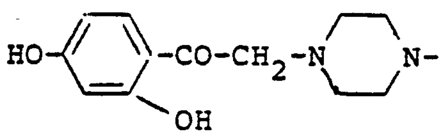 | 150-154°C | 0,16 |
| 6 | 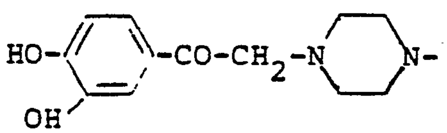 | 210-215°C | 0,08 |
| 7 | 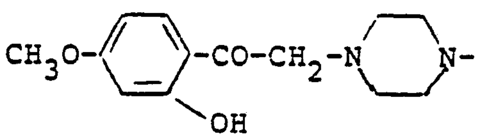 | 224-227°C | 0,32 |
| 8 | 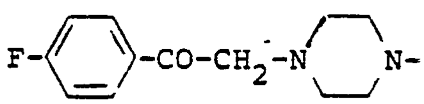 | 168-171°C (Zers.) | 0,33 |
| 9 | 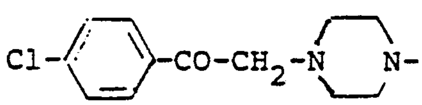 | 197-199°C (Zers.) | 0,32 |

Beispiel 10

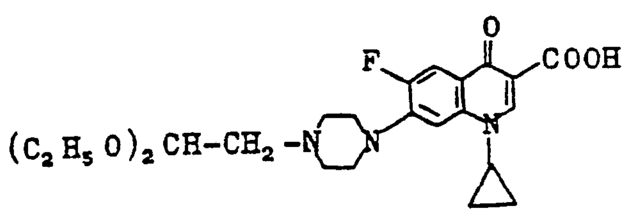

**Beispiel 10**

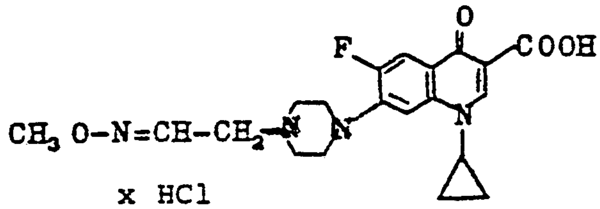

Eine Mischung aus 3,3 g (0,01 Mol) 1-Cyclopropyl-6-fluor-1,4-dihydro-4-oxo-7-(1-piperazinyl)-3-chinolincarbonsäure, 3,85 g (0,02 Mol) Bromacetaldehyddiethylacetal, 2,1 g Triethylamin und 3,35 g Kaliumiodid wird 11 Stunden auf 90°C erhitzt. Die Lösung wird im Hochvakuum eingeengt, mit 20 ml Methanol verrührt und der ausgefallene Niederschlag mehrmals mit Wasser gewaschen und mit Methanol ausgekocht. Ausbeute: 1,3 g (29 %) 1-Cyclopropyl-7-[4-(2,2-diethoxyethyl)-1-piperazinyl]-6-fluor-1,4-di-hydro-4-oxo-3-chinolincarbonsäure mit einem Zersetzungspunkt von 208-212°C; $R_F$-Wert: 0,40.

**Beispiel 11**

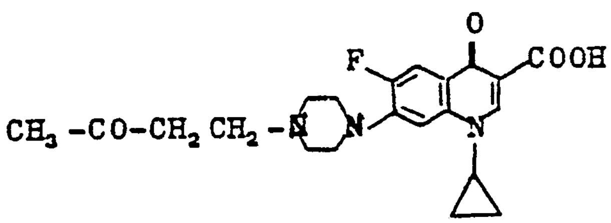

Eine Mischung aus 3,3 g (0,01 Mol) 1-Cyclopropyl-6-fluor-1,4-dihydro-4-oxo-7-(1-piperazinyl)-3-chinolincarbonsäure, 1,6 g Chloracetaldehydoxim-0-methylether und 2,1 g Triethylamin wird 3 Stunden bei 80°C gerührt. Anschließend wird im Hochvakuum eingeengt, mit 30 ml Wasser versetzt und mit 2 n HCl auf pH 2 gestellt. Den Niederschlag saugt man ab, wäscht mit Wasser und Methanol und trocknet im Hochvakuum. Ausbeute: 1,9 g (43 % d.Th.) 1-Cyclopropyl -6-fluor-1,4-dihydro-7-[4-(2-methoximinoethyl)-1-piperazinyl]-4-oxo-3-chinolincarbonsäure-hydrochlorid mit einem Zersetzungspunkt von 215-221°C, R -Wert: 0,29.

NMR ($d_6$-DMSO): 3,87 und 3,88 (2 Singuletts für die $CH_3O$-Gruppen der syn- und anti-Form).

Massenspektrum: $^m/e$ 402, 371, 331, 289, 287, 245 (100%) 229, 70, 56, 44, 32, 31, 27.

**Beispiel 12**

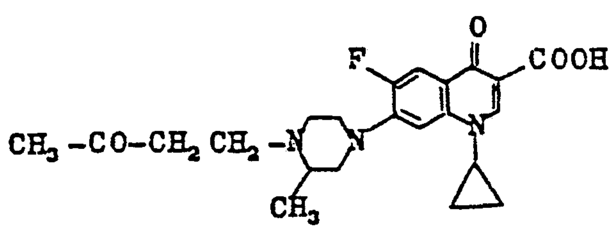

3,31 g (0,01 Mol) 1-Cyclopropyl-6-fluor-1,4-dihydro-4-oxo-7-(1-piperazinyl)-3-chinolincarbonsäure und 3,9 g Methylvinyl-keton werden in 50 ml Ethanol 2 Stunden unter Rückfluß erhitzt. Man saugt ab, wäscht mit Methanol und erhält 2,5 g (62,3 % der Theorie) 1-Cyclopropyl-6-fluor-1,4-dihydro-4-oxo-7-/4-(3-oxobutyl)-1-piperazinyl/-3-chinolincarbonsäure mit einem Zersetzungspunkt von 185-187°C; $R_f$-Wert: 0,14.

**Beispiel 13**

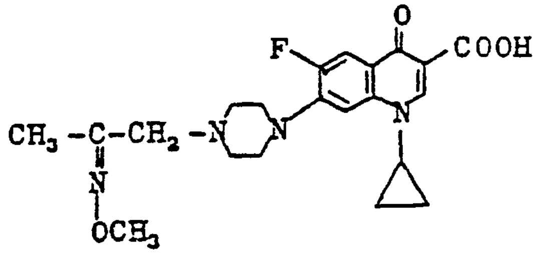

Analog Beispiel 12 erhält man mit dem Ausgangsprodukt aus Beispiel B 1-Cyclopropyl-6-fluor-1,4-dihydro-7-[3-methyl-4-(3-oxobutyl)-1-piperazinyl]-4-oxo-3-chinolincarbonsäure (87 % der Theorie) mit einem Zersetzungspunkt von 176-178° C; $R_f$-Wert: 0,39.

**Beispiel 14**

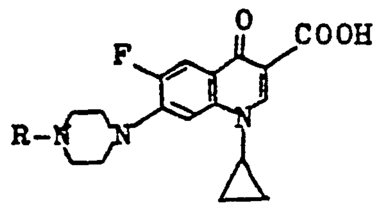

Eine Mischung aus 3,87 g (0,01 Mol) 1-Cyclopropyl-6-fluor-1,4-dihydro-4-oxo-7-[4-(2-oxopropyl)-1-piperazinyl]-3-chinolincarbonsäure und 835 mg (0,01 Mol) Methoxyaminhydrochlorid in 120 ml Ethanol wird mit 0,5 ml konzentrierter Salzsäure versetzt und 3 Stunden unter Rückfluß erhitzt. Die heiße Lösung wird mit etwas "Tonsil" (Bleicherde) verrührt und filtriert. Das nach dem Abkühlen ausfallende Kristallisat wird abgesaugt, mit Ether gewaschen und getrocknet. Ausbeute: 2,1 g (46 % der Theorie) 1-Cyclo-propyl-6-fluor-1,4-dihydro-7-[4-(2-methoximinopropyl)-1-piperazinyl]-4-oxo-3-chinolincarbonsäure mit einem Zersetzungspunkt von 215-217° C; $R_f$-Wert: 0,37.

Analog Beispiel 14 werden die folgenden Verbindungen erhalten:

| Beispiel | R | Schmelzpunkt, °C | $R_f$-Wert |
|---|---|---|---|
| 15 | 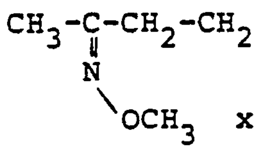 x HCl | 315-320°C (Zers.) | 0,39 |
| 16 | 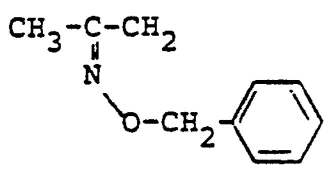 | 184-188°C (Zers.) | 0,40 |
| 17 | 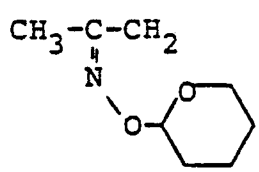 | 105-110°C (Zers.)*) | 0,39 |
| 18 | 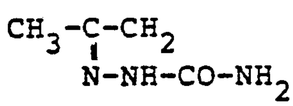 x HCl x $2H_2O$ | 217-219°C (Zers.) | 0,1 |
| 19 | 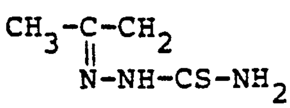 x HCl x $H_2O$ | 219-221°C (Zers.) | 0,24 |

* erstarrter Schaum; z.T. ist der Tetrahydropyranylrest abgespalten.

In der nachstehenden Tabelle sind die minimalen Hemmkonzentrationen (MHK) bei verschiedenen Bakterien für einige der erfindungsgemäßen Verbindungen angegeben.

Minimale Hemmkonzentrationen in mcg / ml im Agarverdünnungstest; Denley-Multipoint-Inokulationsverfahren

| S t a m m | Beispiel 4 | Beispiel 1 | Beispiel 5 | Beispiel 8 | Beispiel 12 | Beispiel 9 |
|---|---|---|---|---|---|---|
| E. coli Neumann | 0,03 | 0,06 | | ≤ 0,015 | ≤ 0,015 | 0,03 |
| E. coli A 261 | ≤ 0,015 | ≤ 0,015 | | ≤ 0,015 | ≤ 0,015 | ≤ 0,015 |
| Klebsiella 8085 | 0,125 | 0,06 | | 0,03 | ≤ 0,015 | 0,125 |
| Klebsiella 6179 | 0,5 | 2 | | 0,25 | 0,25 | 1 |
| Klebsiella 57 USA | 0,06 | 1 | | 0,03 | 0,06 | 0,5 |
| Proteus morganii 932 | ≤ 0,015 | 0,06 | | ≤ 0,015 | ≤ 0,015 | 0,03 |
| Providencia 12052 | 128 | 128 | | 64 | 32 | > 128 |
| Staphylococcus Fk 422 | 2 | 0,25 | 0,25 | 1 | 0,5 | 2 |
| 1756 | 1 | 0,25 | 0,25 | 1 | 0,5 | 2 |
| 133 | 2 | 0,25 | 0,25 | 1 | 0,5 | 2 |

**Patentansprüche:**

1. 1-Cyclopropyl-6-fluor-1,4-dihydro-4-oxo-7-[4-(oxo-alkyl)-1-piperazinyl]-3-chinolincarbonsäuren der Formel (I)

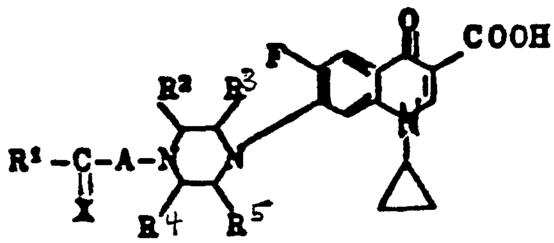

(I)

in der
$R^1$ Wasserstoff, geradkettiges oder verzweigtes Alkyl mit 1 bis 4 Kohlenstoffatomen und gegebenenfalls 1-3 fach durch Trifluormethyl, Methyl, Ethyl, Fluor, Chlor, Brom, Phenyl, Hydroxy-oder Alkoxy mit 1-4 Kohlenstoffatomen substituiertes Phenyl,
$R^2$, $R^3$, $R^4$ und $R^5$ gleich oder verschieden sein können und Wasserstoff Methyl, Ethyl, n- oder i-Propyl,
X O, N-O-R', N-NH-R- und $(OR''')_2$, wobei
R' für Wasserstoff, geradkettiges oder verzweigtes Alkyl mit 1 bis 6 Kohlenstoffatomen, Cycloalkyl mit 5 bis 6 Kohlenstoffatomen, Benzyl, Chlorbenzyl oder Tetrahydropyranyl,
R'' für Methyl, Phenyl, Carbamoyl oder Thiocarbamoyl und
R''' für Methyl und Ethyl oder $(OR''')_2$ für

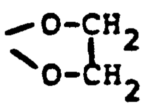

stehen und
A eine Alkylengruppe mit 1 - 4 Kohlenstoffatomen, die gegebenenfalls durch Alkyl mit 1-4 Kohlenstoffatomen oder Phenyl substituiert ist, bedeutet, und deren pharmazeutisch verwendbare Säureadditions-, Alkali- und Erdalkalisalze und Hydrate.

2. 1-Cyclopropyl-6-fluor-1,4-dihydro-4-oxo-7-[4-(oxo-alkyl)-1-piperazinyl]-3-chinolincarbonsäuren der Formel (I)

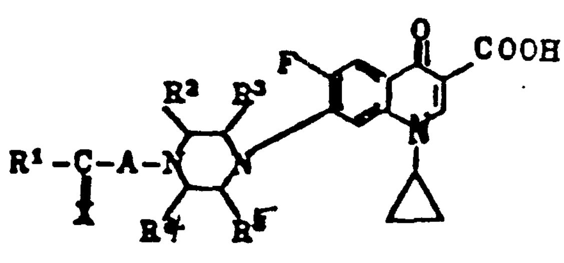

(I)

in der
$R^1$ Wasserstoff, geradkettiges oder verzweigtes Alkyl mit 1-3 Kohlenstoffatomen oder gegebenenfalls 1-3 fach durch Trifluormethyl, Methyl, Ethyl, Fluor, Chlor, Brom, phenyl, Hydroxy oder Alkoxy mit 1-4 Kohlenstoffatomen substituiertes Phenyl,
$R^2$, $R^3$, $R^4$ und $R^5$ Wasserstoff, Methyl oder Ethyl, X O, N-O-R', N-NH-R'' oder $(OR''')_2$ bedeuten, wobei
R' Wasserstoff, Alkyl mit 1-4 Kohlenstoffatomen, Benzyl oder Tetrahydropyranyl,

R'' Carbamoyl oder Thiocarbamoyl,
R''' Methyl oder Ethyl und
A Alkylen mit 1-3 Kohlenstoffatomen
bedeuten, und deren pharmazeutisch verwendbare Säureadditions-, Alkali- und Erdalkalisalze und Hydrate.

3. 1-Cyclopropyl-6-fluor-1,4-dihydro-4-oxo-7-[4-(oxo-alkyl)-1-piperazinyl]-3-chinolincarbonsäuren der Formel (I)

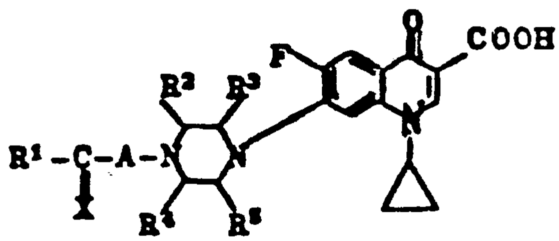

(I)

in der
$R^1$ Wasserstoff, Alkyl mit 1-2 Kohlenstoffatomen oder gegebenenfalls 1-2 fach durch Trifluormethyl, Methyl, Fluor, Chlor, Hydroxy, oder Alkoxy mit 1-2 Kohlenstoffatomen substituiertes Phenyl,
$R^2$ Wasserstoff, Methyl oder Ethyl,
$R^3$ Wasserstoff,
$R^4$ Wasserstoff, Ethyl oder Methyl,
$R^5$ Wasserstoff oder Methyl,
X O, N-O-R', N-NH-R'' oder $(OR''')_2$ bedeuten, wobei
R' Wasserstoff, Alkyl mit 1-2 Kohlenstoffatomen, Benzyl oder Tetrahydropyranyl,
R'' Carbamoyl oder Thiocarbamoyl,
R''' Methyl oder Ethyl und
A Alkylen mit 1-2 Kohlenstoffatomen
bedeuten, und deren pharmazeutisch verwendbare Säureadditions-, Alkali- und Erdalkalisalze und Hydrate.

4. 1-Cyclopropyl-6-fluor-1,4-dihydro-4-oxo-7-[4-(2-oxopropyl)-1-piperazinyl]-3-chinolincarbonsäure.

5. 1-Cyclopropyl-6-fluor-1,4-dihydroxy-4-oxo-7-[4-(4-fluorphenacyl)-1-piperazinyl]-3-chinolincarbonsäure.

6. 1-Cyclopropyl-6-fluor-1,4-dihydro-4-oxo-7-[4-(2-methoxi-minobutyl)-1-piperazinyl]-3-chinolincarbonsäure.

7. 1-Cyclopropyl-6-fluor-1,4-dihydro-4-oxo-7-[4-(3-oxobutyl) -1-pipernzinyl]-3-chinolincarbonsäure.

8) 1-Cyclopropyl-6-fluor-1,4-dihydro-4-oxo-7-[4-(oxoalkyl)-1-piperazinyl]-3-chinolincarbonsäuren der Formel (O)

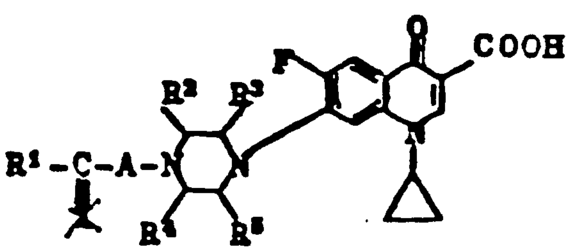

(I)

in der
$R^1$ Wasserstoff, geradkettiges oder verzweigtes Alkyl mit 1 bis 4 Kohlenstoffatomen und gegebenenfalls 1-3 fach durch Trifluormethyl, Methyl, Ethyl, Fluor, Chlor, Brom, Phenyl, Hydroxy oder Alkoxy mit 1-4 Kohlenstoffatomen substituiertes Phenyl,
$R^2$, $R^3$, $R^4$ und $R^5$ gleich oder verschieden sein können und Wasserstoff, Methyl, Ethyl, n- oder i-Propyl,
X O, N-O-R', N-NH-R'' und $(OR''')_2$,
wobei
R' für Wasserstoff, geradkettiges oder verzweigtes Alkyl mit 1 bis 6 Kohlenstoffatomen, Cycloalkyl mit 5 bis 6

Kohlenstoffatomen, Benzyl, Chlorbenzyl oder Tetrahydropyranyl,
R'' für Methyl, Phenyl, Carbamoyl oder Thiocarbamoyl und
R''' für Methyl und Ethyl oder $(OR-)_2$ für

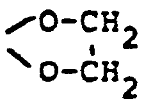

stehen und
A eine gegebenenfalls durch Alkyl mit 1-4 Kohlenstoffatomen oder Phenyl substituierte Alkylenkette mit 1 bis 4 Kohlenstoffatomen bedeuten, und deren pharmazeutisch verwendbare Säureadditions-, Alkali- und Erdalkalisalze und Hydrate zur Behandlung bakterieller Erkrankungen.

9) Verfahren zur Herstellung von 1-Cyclopropyl-6-fluor-1,4-dihydro-4-oxo-7-[4-(oxoalkyl)-1-piperazinyl]-3-chinolincarbonsäuren der Formel (I) in Anspruch 1, dadurch gekennzeichnet, daß man entweder

a) Verbindungen der Formel (II)

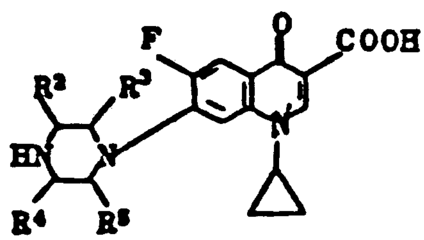

(II)

in welcher $R^2$, $R^3$, $R^4$ und $R^5$ die oben angegebene Bedeutung haben, mit einer Verbindung der Formel (III)

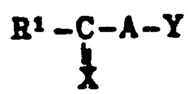

(III)

in welcher
$R^1$, X und A die oben angegebenen Bedeutungen haben und Y für Halogen, vorzugsweise Chlor oder Brom steht, umsetzt, oder

b) Verbindungen der Formel (II)

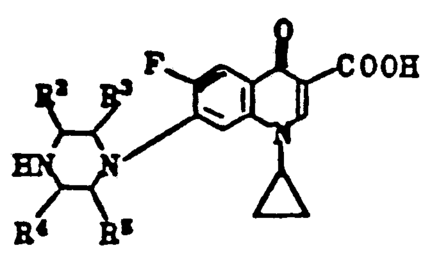

(II)

in welcher
$R^2$, $R^3$, $R^4$ und $R^5$ die oben angegebene Bedeutungen haben, mit Alkenonen der Formel (IV)

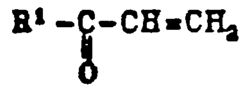

(IV)

in welcher

$R^1$ die oben angegebene Bedeutung hat, zu den erfindungsgemäßen Verbindungen der Formel (Ia) (I; mit X = O, A = $-CH_2CH_2-$)

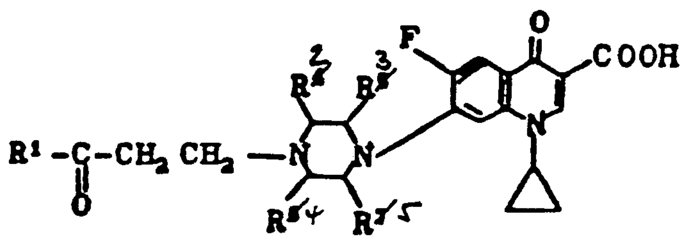

(Ia)

umsetzt, oder

c) Verbindungen der Formel (V)

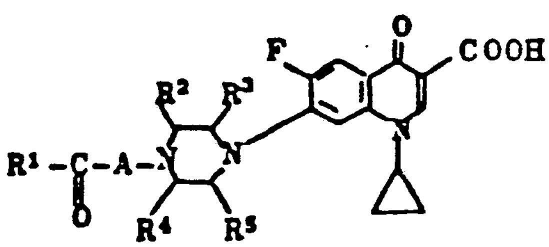

(V)

in welcher $R^1$, $R^2$, $R^3$, $R^4$, $R^5$ und A die oben anegebene Bedeutung haben, mit Verbindungen der Formel (VIa) beziehungsweise (VIb)

$H_2H-O-R'$ (VIa)

$H_2N-NH-R''$ (VIb),

in welcher

R' und R'' die oben angegebenen Bedeutungen besitzen, bzw. deren Hydrochloriden oder Sulfaten umsetzt.

10. Verwendung von 1-Cyclopropyl-6-flour-1,4-dihydro-4-oxo-7-[4-(oxoalkyl) -1-piperazinyl]-3-chinolincarbonsäuren der Formel (I)

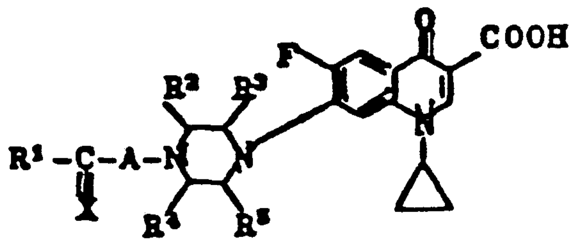

(I)

in der

$R^1$ Wasserstoff, geradkettiges oder verzweigtes Alkyl mit 1 bis 4 Kohlenstoffatomen und gegebenenfalls 1-3 fach durch Trifluormethyl, Methyl, Ethyl, Fluor, Chlor, Brom, Phenyl, Hydroxy oder Alkoxy mit 1-4 Kohlenstoffatomen substituiertes Phenyl,

$R^2$, $R^3$, $R^4$ und $R^5$ gleich oder verschieden sein können und Wasserstoff Methyl, Ethyl, n- oder i-Propyl,

O, N-O-R', N-NH-R'' und $(OR''')_2$, wobei

R' für Wasserstoff, geradkettiges oder verzweigtes Alkyl mit 1 bis 6 Kohlenstoffatomen, Cycloalkyl mit 5 bis 6 Kohlenstoffatomen, Benzyl, Chlorbenzyl oder Tetrahydropyranyl,

R'' für Methyl, Phenyl, Carbamoyl oder Thiocarbamoyl und

R''' für Methyl und Ethyl oder $(OR''')_2$ für

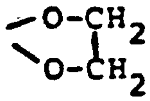

stehen und

A eine Alkylengruppe mit 1 - 4 Kohlenstoffatomen, die gegebenenfalls durch Alkyl mit 1-4 Kohlenstoffatomen oder Phenyl substituiert ist, bedeutet,

und deren pharmazeutisch verwendbaren Säureadditions-, Alkali- und Erdalkalisalzen und Hydraten zur Herstellung von Arzneimitteln.

**Claims**

1. 1-Cyclopropyl-6-fluoro-1,4-dihydro-4-oxo-7-[4-(oxoalkyl)-1-piperazinyl]-3-quinoline- carboxylic acids of the formula (I)

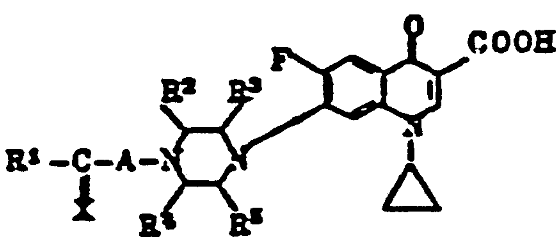

(I)

in which

$R^1$ denotes hydrogen, straight-chain or branched alkyl having 1 to 4 carbon atoms and phenyl which is optionally substituted 1-3 times by trifluoromethyl, methyl, ethyl, fluorine, chlorine, bromine, phenyl, hydroxyl or alkoxy having 1-4 carbon atoms,

$R^2$, $R^3$, $R^4$ and $R^5$ can be identical or different and denote hydrogen, methyl, ethyl, n- or ipropyl,

X denotes O, N-O-R', N-NH-R'' and $(OR''')_2$, in which
R' represents hydrogen, straight-chain or branched alkyl having 1 to 6 carbon atoms, cycloalkyl having 5 to 6 carbon atoms, benzyl, chlorobenzyl or tetrahydropyranyl,
R'' represents methyl, phenyl, carbamoyl or thiocarbamoyl and
R''' represents methyl and ethyl or $(OR''')_2$ represents

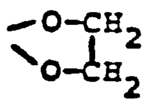

and
A denotes an alkylene group having 1 - 4 carbon atoms, which is optionally substituted by alkyl having 1-4 carbon atoms or phenyl,
and their pharmaceutically utilisable acid addition, alkali metal and alkaline earth metal salts and hydrates.

2. 1-Cyclopropyl-6-fluoro-1,4-dihydro-4-oxo-7-[4-(oxoalkyl)-1-pipcrazinyl] -3-quinoline-carboxylic acids of the formula (I)

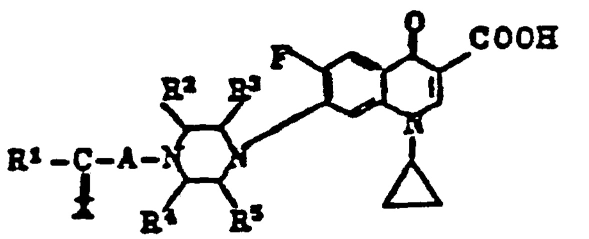

(I)

in which
$R^1$ denotes hydrogen, straight-chain or branched alkyl having 1-3 carbon atoms or phenyl which is optionally substituted 1-3 times by trifluoromethyl, methyl, ethyl, fluorine, chlorine, bromine, phenyl, hydroxyl or alkoxy having 1-4 carbon atoms,
$R^2$, $R^3$, $R^4$ and $R^5$ denote hydrogen, methyl or ethyl,
X denotes O, N-O-R', N-NH-R'' or $(OR''')_2$, in which R' denotes hydrogen, alkyl having 1-4 carbon atoms, benzyl or tetrahydropyranyl,
R'' denotes carbamoyl or thiocarbamoyl,
R''' denotes methyl or ethyl and
A denotes alkylene having 1-3 carbon atoms, and their pharmaceutically utilisable acid addition, alkali metal and alkeline earth metal salts and hydrates.

3. 1-Cyclopropyl-6-fluoro-1,4-dihydro-4-oxo-7-[4-(oxoalkyl)-1-piperazinyl]-3-quinoline- carboxylic acids of the formula (I)

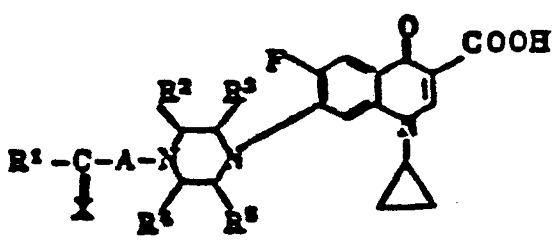

(I)

in which
$R^1$ denotes hydrogen, alkyl having 1-2 carbon atoms or phenyl which is optionally substituted 1-2 times by trifluoromethyl, methyl, fluorine, chlorine, hydroxyl or alkoxy having 1-2 carbon atoms,

$R^2$ denotes hydrogen, methyl or ethyl,
$R^3$ denotes hydrogen,
$R^4$ denotes hydrogen, ethyl or methyl,
$R^5$ denotes hydrogen or methyl,
X denotes O, N-O-R', N-NH-R'' or $(OR''')_2$, in which
R' denotes hydrogen, alkyl having 1-2 carbon atoms, benzyl or tetrahydropyranyl,
R'' denotes carbamoyl or thiocarbamoyl,
R''' denotes methyl or ethyl and
A denotes alkylene having 1-2 carbon atoms, and their pharmaceutically utilisable acid addition, alkali metal and alkaline earth metal salts and hydrates.

4. 1-Cyclopropyl-6-fluoro-1,4-dihydro-4-oxo-7-[4-C2-oxopropyl)-1-piperazinyl] -3-quinoline-carboxylic acid.

5. 1-Cyclopropyl-6-fluoro-1,4-dihydro-4-oxo-7-[4-C4-fluorophenacyl)-1-piperazinyl] -3-quinoline-carboxylic acid.

6. 1-Cyclopropyl-6-fluoro-1,4-dihydro-4-oxo-7-[4-(2-methoximinobutyl)-1-piperazinyl]-3-quinoline-carboxylic acid.

7. 1-Cyclopropyl-6-fluoro-1,4-dihydro-4-oxo-7-[4-(3-oxobutyl)-1-piperazinyl]-3-quinoline-carboxylic acid.

8. 1-Cyclopropyl-6-fluoro-1 4-dihydro-4-oxo-7-[4-(oxoalkyl)-1-pipcrazinyl]-3-quinoline- carboxylic acids of the formula (I)

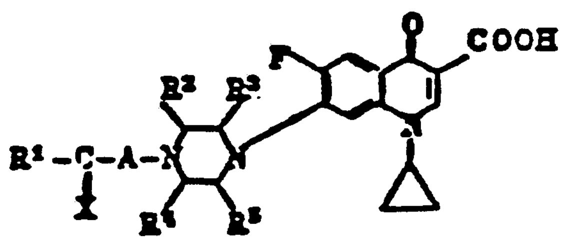

(I)

in which
$R^1$ denotes hydrogen, straight-chain or branched alkyl having 1 to 4 carbon atoms and phenyl which is optionally substituted 1-3 times by trifluoromethyl, methyl, ethyl, fluorine, chlorine, brominc, phenyl, hydroxyl or alkoxy having 1-4 carbon atoms,
$R^2$, $R^3$, $R^4$ and $R^5$ can be identical or different and denote hydrogen, methyl, ethyl, n- or i-propyl,
X denotes O, N-O-R', N-NH-R'' and $(OR''')_2$, in which
R' represents hydrogen, straight-chain or branched alkyl having 1 to 6 carbon atoms, cycloalkyl having 5 to 6 carbon atoms, benzyl, chlorobenzyl or tetrahydropyranyl,
R'' represents methyl, phenyl, carbamoyl or thiocarbamoyl and
R''' represents methyl and ethyl or $(OR''')_2$ represents

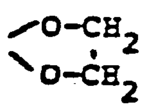

and
A denotes an alkylene chain having 1 to 4 carbon atoms, which is optionally substituted by alkyl having 1-4 carbon atoms or phenyl,
and their pharmaceutically utilisable acid addition, alkali metal and alkaline earth metal salts and hydrates for the treatment of bacterial diseases.

9. Process for the preparation of 1-cyclopropyl-6-fluoro-1,4-dihydro-4-oxo-7-[4-Coxoalkyl)-i-piperazinyl]-3-quinoline-carboxylic acids of the formula(I)in Claim 1, characterised in that either

a) compounds of the formula (II)

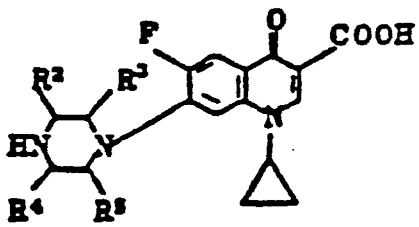

(II)

in which
$R^2$, $R^3$, $R^4$ and $R^5$ have the meaning indicated above,
are reacted with a compound of the formula (III)

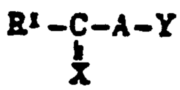

(III)

in which
$R^1$, X and A have the meanings indicated above, and
Y represents halogen, preferably chlorine or bromine,
or
b) compounds of the formula (II)

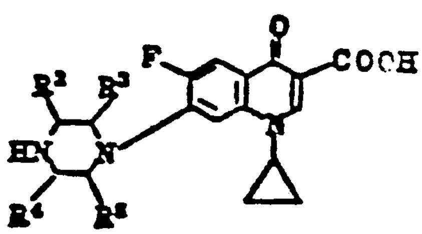

(II)

in which
$R^2$, $R^3$, $R^4$ and $R^5$ have the meanings indicated above,
are reactcd with alkenones of the formula (IV)

(IV)

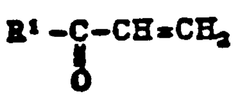

in which
$R^1$ has the meaning indicated above, to give the compounds of the formula (Ia) = (I; with X = O, A = -$CH_2CH_2$-) according to the invention

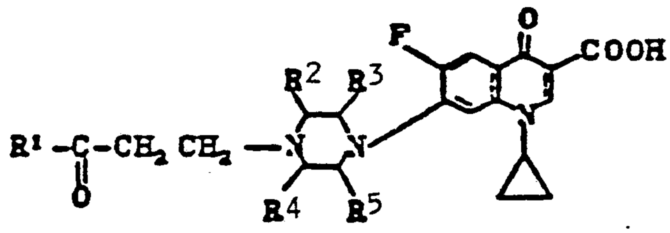

(Ia)

or

c) compounds of the formula (V)

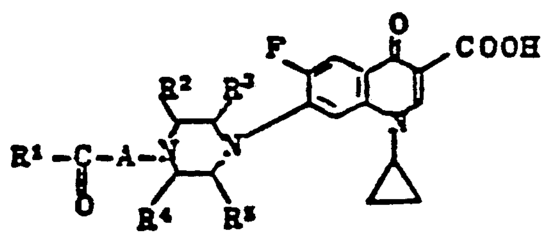

(V)

in which

$R^1$, $R^2$, $R^3$, $R^4$, $R^5$ and A have the meaning indicated above,

are reacted with compounds of the formula (VIa) or (VIb)

$H_2N$-O-R′ (VIa)

$H_2N$-NH-R″ (VIb)

in which

R′ and R″ have the meanings indicated above, or their hydrochlorides or sulphates.

10. Use of 1-cyclopropyl-6-fluoro-1,4- dihydro-4-oxo-7-[4-(oxoalkyl)-1-piperazinyl]-3-quinoline- carboxylic acids of the formula (I)

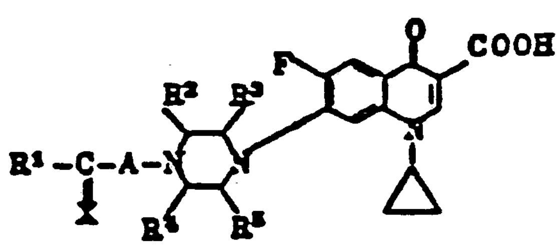

(I)

in which

$R^1$ denotes hydrogen, straight-chain or branched alkyl having 1 to 4 carbon atoms and phenyl which is optionally substituted 1-3 times by trifluoromethyl, methyl, ethyl, fluorine, chlorine, bromine, phenyl, hydroxyl or alkoxy having 1-4 carbon atoms,

$R^2$, $R^3$, $R^4$ and $R^5$ can be identical or different and denote hydrogen, methyl, ethyl, n- or ipropyl,

O, N-O-R′, N-NH-R″ and $(OR''')_2$,

in which

R' represents hydrogen, straight-chain or branched alkyl having 1 to 6 carbon atoms, cycloalkyl having 5 to 6 carbon atoms, benzyl, chlorobenzyl or tetrahydropyranyl,

R'' represents methyl, phenyl, carbamoyl or thiocarbamoyl and

R''' represents methyl and ethyl or $(OR''')_2$ represents

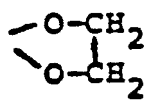

and

A denotes an alkylene group having 1 - 4 carbon atoms, which is optionally substituted by alkyl having 1-4 carbon atoms or phenyl,

and their pharmaceutically utilisable acid addition, alkali metal and alkaline earth metal salts and hydrates. for the production of medicaments.

**Revendications**

1. Acides 1-cyclopropyl-6-fluoro-1,4-dihydro-4-oxo-7-[4-(oxoalkyl)-1-pipérazinyl]-3-quinoléinecarboxy-liques de formule (I)

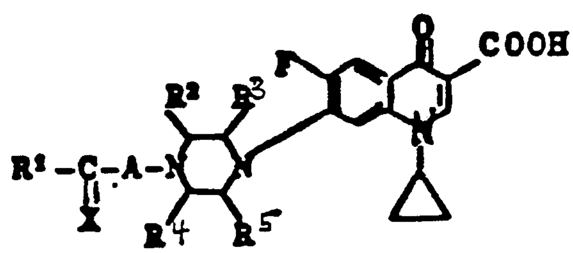

(I)

dans laquelle

$R^1$ représente l'hydrogène, un groupe alkyle à chaîne droite ou ramifié ayant 1 à 4 atomes de carbone et un groupe phényle éventuellement substitué 1 à 3 fois par des substituants trifluorométhyle, méthyle, éthyle, fluoro, chloro, bromo, phényle, hydroxy ou alkoxy ayant 1 à 4 atomes de carbone,

$R^2$, $R^3$, $R^4$ et $R^5$ peuvent être identiques ou différents et représentent de l'hydrogène, un groupe méthyle, éthyle, n-propyle ou isopropyle,

X représente O, N-O-R', N-NH-R'' et $(OR''')_2$,

R' représentant l'hydrogène, un groupe alkyle à chaîne droite ou ramifié ayant 1 à 6 atomes de carbone, cycloalkyle ayant 5 ou 6 atomes de carbone, benzyle, chlorobenzyle ou tétrahydropyrannyle,

R'' étant un groupe méthyle, phényle, carbamoyle ou thiocarbamoyle et

R''' désignant les groupes méthyle et éthyle ou bien $(OR''')_2$ étant un groupe

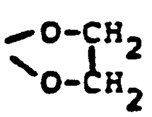

et

A étant un groupe alkylène ayant 1 à 4 atomes de carbone qui éventuellement substitué par un radical alkyle ayant 1 à 4 atomes de carbone ou phényle,

et leurs sels d'addition d'acides pharmaceutiquement acceptables, leurs sels alcalins et alcalino-terreux et leurs hydrates.

2. Acides 1-cyclopropyl-6-fluoro-1,4-dihydro-4-oxo-7-[4-(oxoalkyl)-1-pipérazinyl]-3-quinoléinecarboxy-liques de formule (I)

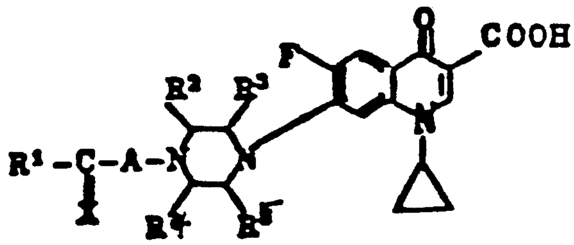

(I)

dans laquelle

R[1] représente l'hydrogène, un groupe alkyle à chaîne droite ou ramifié ayant 1 à 3 atomes de carbone ou un groupe phényle éventuellement substitué 1 à 3 fois par des substituants trifluorométhyle, méthyle, éthyle, fluoro, chloro, bromo, phényle, hydroxy ou alkoxy ayant 1 à 4 atomes de carbone,

$R^2$, $R^3$, $R^4$ et $R^5$ représentent l'hydrogène, le groupe méthyle ou le groupe éthyle,

X représente O, N-NH-R'' ou $(OR''')_2$,

R' représentant l'hydrogène, un groupe alkyle ayant 1 à 4 atomes de carbone, benzyle ou tétrahydropyrannyle,

R'' étant un groupe carbamoyle ou thiocarbamoyle,

R''' étant un groupe méthyle ou éthyle et

A étant un groupe alkylène ayant 1 à 3 atomes de carbone,

et leurs sels d'addition d'acides pharmaceutiquement acceptables, leurs sels alcalins et alcalinoterreux et leurs hydrates.

3. Acides 1-cyclopropyl-6-fluoro-1,4-dihydro-4-oxo-7-[(oxoalkyl)-1-pipérazinyl]-3-quinoléinecarboxy-liques de formule (I)

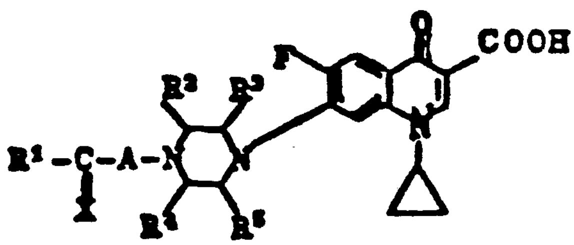

(I)

dans laquelle

$R^1$ représente l'hydrogène, un groupe alkyle ayant 1 ou 2 atomes de carbone, ou un groupe phényle éventuellement substitué une ou deux fois par des substituants trifluorométhyle, méthyle, fluoro, chloro, hydroxy ou alkoxy ayant 1 à 2 atomes de carbone,

$R^2$ est l'hydrogène, le groupe méthyle ou le groupe éthyle,

$R^3$ est l'hydrogène,

$R^4$ est l'hydrogène, le groupe éthyle ou le groupe méthyle,

$R^5$ est l'hydrogène ou le groupe méthyle

X représente O, N-O-R', N-NH-R'' ou $OR''')_2$,

R' étant un atome d'hydrogène, un groupe alkyle ayant 1 ou 2 atomes de carbone, benzyle ou tétrahydropyrannyle,

R'' représentant un groupe carbamoyle ou thiocarbamoyle,

R''' étant un groupe méthyle ou éthyle et

A étant un groupe alkylène de 1 ou 2 atomes de carbone,

et leurs sels d'addition d'acides pharmaceutiquement acceptables, leurs sels de métaux alcalins et alcalinoterreux et leurs hydrates.

4. L'acide 1-cyclopropyl-6-fluoro-1,4-di-hydro-4-oxo-7-[4-(2-oxopropyl)-1-pipérazinyl]-3-quinoléine-carboxylique.

5. L'acide 1-cyclopropyl-6-fluoro-1,4-dihydroxy-4-oxo-7-[4(4fluorophénacyl)-1-pipérazinyl45A-3-quinoléine-carboxylique.

6. L'acide 1-cyclopropyl-6-fluoro-1,4-dihydro-4-oxo-7-[4-(2-méthoxyminobutyl)-1-pipérazinyl]-3-quinoléinecarboxylique.

7. L'acide 1-cyclopropyl-6-fluoro-1,4-dihydro-4-oxo-7-[4-(3-oxobutyl)-1-pipérazinyl]-3-quinoléine-carboxylique.

8. Acides 1-cyclopropyl-6-fluoro-1,4-dihydro-4-oxo-7-[4-(oxoalkyl)-1-pipérazinyl]-3-quinoléinecarboxy-liques de formule (I)

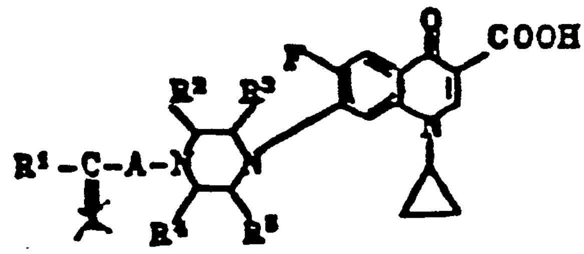

(I)

dans laquelle

$R^1$ représente l'hydrogène, un groupe alkyle à chaîne droite ou ramifié ayant 1 à 4 atomes de carbone et un groupe phényle éventuellement substitué 1 à 3 fois par des substituants trifluorméthyle, méthyle, éthyle, fluoro, chloro, bromo, phényle, hydroxy ou alkoxy ayant 1 à 4 atomes de carbone,

$R^2$, $R^3$, $R^4$ et $R^5$ peuvent être identiques ou différents et représentent de l'hydrogène, un groupe méthyle, éthyle, n-propyle ou isopropyle,

X représente O, N-O-R', N-NH-R'' et $(OR''')_2$,

R' représentant l'hydrogène, un groupe alkyle à chaîne droite ou ramifié ayant 1 à 6 atomes de carbone, cycloalkyle ayant 5 ou 6 atomes de carbone, benzyle, chlorobenzyle ou tétrahydropyrannyle,

R'' étant un groupe méthyle, phényle, carbamoyle ou thiocarbamoyle et

R''' désignant les groupes méthyle et éthyle ou bien $(OR''')_2$ étant un groupe

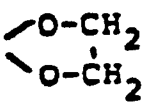

et

A étant un groupe alkylène ayant 1 à 4 atomes de carbone qui est éventuellement substitué par un radical alkyle ayant 1 à 4 atomes de carbone ou phényle,

et leur sels d'addition d'acides pharmaceutiquement acceptables, leurs hydrates pour le traitement de maladiesbactériennes.

9. Procédé de production d'acides 1-cyclopro-pyl-6-fluoro-1,4-dihydro-4-oxo-7[4-(oxoalkyl)-1-pipéra-zinyl]-3-quinoléinecarboxyliques de formule (I) suivant la revendication 1, caractérisé en ce que

a) on fait réagir les composés de formule (II)

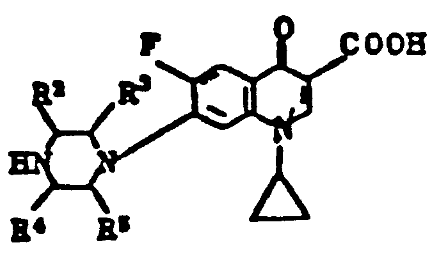

(II)

dans laquelle $R^2$, $R^3$, $R^4$ et $R^5$ ont la définition indiquée ci-dessus, avec un composé de formule (III)

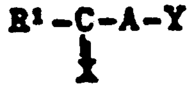

(III)

dans laquelle

$R^1$, X et A ont les définitions indiquées ci-dessus et Y désigne un halogène, de préférence le chlore ou le brome, ou bien

b) on fait réagir des composés de formule (II)

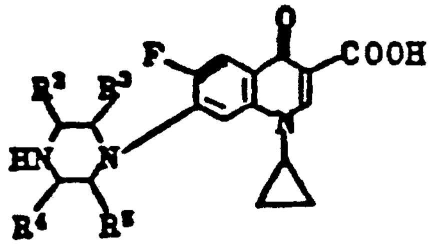

(II)

dans laquelle

$R^2$, $R^3$, $R^4$ et $R^5$ ont les définitions indiquées cidessus, avec des alcénones de formule (IV)

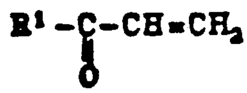

(IV)

dan laquelle

$R^1$ a la définition indiquée ci-dessus pour former les composés conformes à l'invention de formule (ia) = (I; avec X = O, A = $CH_2CH_2$-)

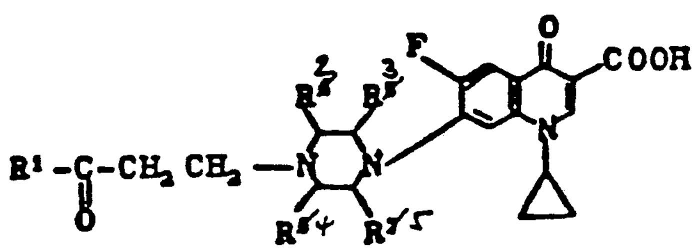

(Ia)

ou bien

c) on fait réagir des composés de formule (V)

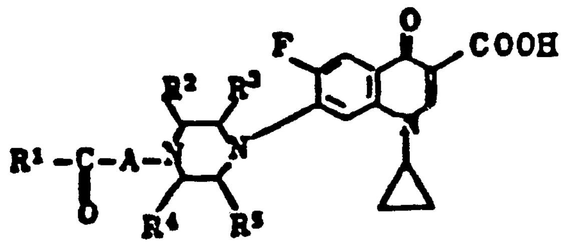

(V)

dans laquelle $R^1$, $R^2$, $R^3$, $R^4$, $R^5$ et A ont la définition indiquée ci-dessus, avec des composés de formule (VIa) et respectivement (VIb)

$$H_2N-O-R' \qquad (VIa)$$

$$H_2N-NH-R'' \qquad (VIb)$$

dans lesquelles

R' et R'' ont les définitions indiquées ci-dessus, ou leurs chlorhydrates ou leurs sulfates.

10. Utilisation d'acides 1-cyclopropyl-6-fluoro-1,4-dihydro-4-oxo-7-[-(oxoalkyl)-1-pipérazinyl]-3-quinoléinecarboxyliques de formule (I)

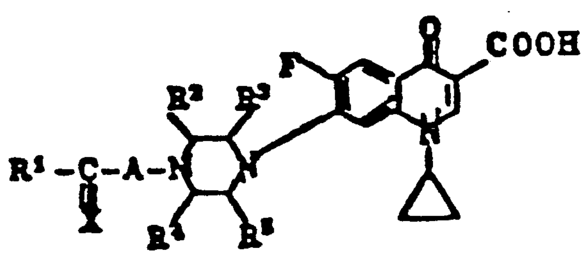

(I)

dans laquelle

$R^1$ représente l'hydrogène, un groupe alkyle à chaîne droite ou ramfié ayant 1 à 4 atomes de carbone et un groupe phényle éventuellement substitué 1 à 3 fois par des substituants trifluorométhyle, méthyle, éthyle, fluoro, chloro, bromo, phényle, hydroxy ou alkoxy ayant 1 à 4 atomes de carbone,

$R^2$, $R^3$, $R^4$ et $R^5$ peuvent être identiques ou différents et représentent de l'hydrogène, un groupe méthyle, éthyle, n-propyle ou isopropyle,

O, N-O-R', N-NH-R'' et $(OR''')_2$,

R' représentant l'hydrogène, un groupe alkyle à chaîne droite ou ramifié ayant 1 à 6 atomes de carbone, cycloalkyle ayant 5 ou 6 atomes de carbone, benzyle, chlorobenzyle ou tétrahydropyrannyle,

R'' étant un groupe méthyle, phényle, carbamoyle ou thiocarbamoyle et

R''' désignant les groupes méthyle et éthyle ou bien $(OR''')_2$ étant un groupe

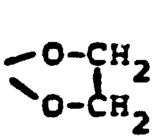

et

A étant un groupe alkylène ayant 1 à 4 atomes de carbone qui est éventuellement substitué par un radical alkyle

ayant 1 à 4 atomes de carbone ou phényle,

et de leurs sels d'addition d'acides pharmaceutiquement utilisables, de leurs sels alcalins et alcalino-terreux et de leurs hydrates pour la préparation de médicaments.